# EUROPEAN PATENT APPLICATION

(11) **EP 3 228 630 A1**
(43) Date of publication of application: **11.10.2017**
(21) Application number: 16164235.0
(22) Date of filing: 07.04.2016
(51) Int. Cl.: C07K 14/515, A61K 38/17, C07K 16/28, A61K 39/00

(54) **COMBINATION OF AN APELIN ANTAGONIST AND AN ANGIOGENESIS INHIBITOR FOR THE TREATMENT OF CANCER**

(71) Applicant: IMBA-Institut für Molekulare Biotechnologie GmbH, 1030 Wien (AT)
(72) Inventor: PENNINGER, Josef, 1130 Vienna (AT); URIBESALGO, Iris, 1160 Vienna (AT)
(74) Representative: Sonn & Partner Patentanwälte

(57) **Abstract**

The field of the present invention is the field of anti-angiogenic cancer therapy. Efforts are needed to reduce or overcome resistance of cancer to anti-angiogenic therapies, such as therapies based on the receptor tyrosine kinase (RTK) inhibitor sunitinib. It is hence an object of the present invention to provide an agent that can be combined with an anti-angiogenic therapy in order to reduce or overcome resistance to the anti-angiogenic therapy. The present invention thus relates to a pharmaceutical composition or set comprising an apelin antagonist and/or apelin receptor antagonist, and an angiogenesis inhibitor. The angiogenesis inhibitor is an RTK inhibitor, preferably sunitinib. The apelin antagonist can be an anti-apelin antibody. The apelin receptor antagonist can be an apelin peptide analogue or an anti-apelin-receptor antibody. Furthermore, methods of cancer treatment, especially breast or lung cancer treatment, based on an apelin antagonist and/or apelin receptor antagonist, and an angiogenesis inhibitor are disclosed.

SEQUENCE LISTING

<110> IMBA - INSTITUT FÃ\234R MOLEKULARE BIOTECHNOLOGIE GMBH

<120> Combination of an apelin antagonist and an angiogenesis inhibitor for
the treatment of cancer

<130> R66032

<160> 23

<170> BiSSAP 1.3

<210> 1
<211> 77
<212> PRT
<213> Homo sapiens

<220>
<223> apelin-77

<400> 1
Met Asn Leu Arg Leu Cys Val Gln Ala Leu Leu Leu Leu Trp Leu Ser
1               5                   10                  15
Leu Thr Ala Val Cys Gly Gly Ser Leu Met Pro Leu Pro Asp Gly Asn
            20                  25                  30
Gly Leu Glu Asp Gly Asn Val Arg His Leu Val Gln Pro Arg Gly Ser
        35                  40                  45
Arg Asn Gly Pro Gly Pro Trp Gln Gly Gly Arg Arg Lys Phe Arg Arg
    50                  55                  60
Gln Arg Pro Arg Leu Ser His Lys Gly Pro Met Pro Phe
65                  70                  75

<210> 2
<211> 55
<212> PRT
<213> Homo sapiens

<220>
<223> apelin-55

<400> 2
Gly Ser Leu Met Pro Leu Pro Asp Gly Asn Gly Leu Glu Asp Gly Asn
1               5                   10                  15
Val Arg His Leu Val Gln Pro Arg Gly Ser Arg Asn Gly Pro Gly Pro
            20                  25                  30
Trp Gln Gly Gly Arg Arg Lys Phe Arg Arg Gln Arg Pro Arg Leu Ser
        35                  40                  45
His Lys Gly Pro Met Pro Phe
    50                  55

<210> 3
<211> 36
<212> PRT
<213> Homo sapiens

<220>
<223> apelin-36

<400> 3
Leu Val Gln Pro Arg Gly Ser Arg Asn Gly Pro Gly Pro Trp Gln Gly
1               5                   10                  15
Gly Arg Arg Lys Phe Arg Arg Gln Arg Pro Arg Leu Ser His Lys Gly

20                  25                  30
Pro Met Pro Phe
        35

<210> 4
<211> 17
<212> PRT
<213> Homo sapiens

<220>
<223> apelin-17

<400> 4
Lys Phe Arg Arg Gln Arg Pro Arg Leu Ser His Lys Gly Pro Met Pro
1               5                   10                  15
Phe


<210> 5
<211> 13
<212> PRT
<213> Homo sapiens

<220>
<221> MOD_RES
<222> 1
<223> PYRROLIDONE CARBOXYLIC ACID

<220>
<223> pyr-apelin-13

<400> 5
Gln Arg Pro Arg Leu Ser His Lys Gly Pro Met Pro Phe
1               5                   10

<210> 6
<211> 13
<212> PRT
<213> Homo sapiens

<220>
<223> apelin-13

<400> 6
Gln Arg Pro Arg Leu Ser His Lys Gly Pro Met Pro Phe
1               5                   10

<210> 7
<211> 12
<212> PRT
<213> Homo sapiens

<220>
<223> apelin-12

<400> 7
Arg Pro Arg Leu Ser His Lys Gly Pro Met Pro Phe
1               5                   10

<210> 8
<211> 21
<212> PRT
<213> Homo sapiens

<220>
<223> apelin epitope

<400> 8
Val Gln Pro Arg Gly Ser Arg Asn Gly Pro Gly Pro Trp Gln Gly Gly
1               5                   10                  15
Arg Arg Lys Phe Arg
            20

<210> 9
<211> 12
<212> PRT
<213> Homo sapiens

<220>
<223> apelin epitope

<400> 9
Arg Gly Ser Arg Asn Gly Pro Gly Pro Trp Gln Gly
1               5                   10

<210> 10
<211> 7
<212> PRT
<213> Homo sapiens

<220>
<223> apelin epitope

<400> 10
Arg Asn Gly Pro Gly Pro Trp
1               5

<210> 11
<211> 11
<212> PRT
<213> Homo sapiens

<220>
<223> apelin epitope

<400> 11
Pro Arg Leu Ser His Lys Gly Pro Met Pro Phe
1               5                   10

<210> 12
<211> 7
<212> PRT
<213> Homo sapiens

<220>
<223> apelin epitope

<400> 12
Pro Arg Leu Ser His Lys Gly
1               5

<210> 13
<211> 8
<212> PRT
<213> Homo sapiens

<220>
<223> apelin epitope

<400> 13
Leu Ser His Lys Gly Pro Met Pro
1               5

<210> 14
<211> 13
<212> PRT
<213> Homo sapiens

<220>
<223> apelin F13A

<400> 14
Gln Arg Pro Arg Leu Ser His Lys Gly Pro Met Pro Ala
1               5                   10

<210> 15
<211> 26
<212> PRT
<213> Homo sapiens

<220>
<223> apelin receptor epitope

<400> 15
Met Glu Glu Gly Gly Asp Phe Asp Asn Tyr Tyr Gly Ala Asp Asn Gln
1               5                   10                  15
Ser Glu Cys Glu Tyr Thr Asp Trp Lys Ser
            20                  25

<210> 16
<211> 20
<212> PRT
<213> Homo sapiens

<220>
<223> apelin receptor epitope

<400> 16
Gly Gly Asp Phe Asp Asn Tyr Tyr Gly Ala Asp Asn Gln Ser Glu Cys
1               5                   10                  15
Glu Tyr Thr Asp
            20

<210> 17
<211> 15
<212> PRT
<213> Homo sapiens

<220>
<223> apelin receptor epitope

<400> 17
Asn Tyr Tyr Gly Ala Asp Asn Gln Ser Glu Cys Glu Tyr Thr Asp
1               5                   10                  15

<210> 18
<211> 10
<212> PRT
<213> Homo sapiens

<220>
<223> apelin receptor epitope

<400> 18
Asp Asn Gln Ser Glu Cys Glu Tyr Thr Asp
1               5                   10

<210> 19
<211> 7
<212> PRT
<213> Homo sapiens

<220>
<223> apelin receptor epitope

<400> 19
Ser Glu Cys Glu Tyr Thr Asp
1               5

<210> 20
<211> 8
<212> PRT
<213> Homo sapiens

<220>
<223> apelin receptor epitope

<400> 20
Gly Asp Leu Glu Asn Thr Thr Lys
1               5

<210> 21
<211> 6
<212> PRT
<213> Homo sapiens

<220>
<223> apelin receptor epitope

<400> 21
Asp Leu Glu Asn Thr Thr
1               5

<210> 22
<211> 9
<212> PRT
<213> Artificial Sequence

<220>
<223> ALX40-4C

<400> 22
Arg Arg Arg Arg Arg Arg Arg Arg Arg
1               5

<210> 23
<211> 14
<212> PRT
<213> Artificial Sequence

<220>
<223> cyclo(1-6)CRPRLC-KH-cyclo(9-14)CRPRLC

<400> 23
Cys Arg Pro Arg Leu Cys Lys His Cys Arg Pro Arg Leu Cys
1               5                   10

## Description

### Field of the invention

The field of the present invention is the field of anti-angiogenic cancer therapy, especially anti-angiogenic breast or lung cancer therapy.

### Background of the invention

Cancer is the second leading cause of mortality in Europe. Activating angiogenesis, the sprouting of new blood vessels from the existing vasculature, is one of the hallmarks of cancer and facilitates rapid tumour growth and metastasis (Hanahan and Weinberg, 2011). Thus, inhibiting tumour neo-angiogenesis is a strategy for cancer treatment currently being pursued.

Anti-angiogenic treatments targeting vascular endothelial growth factor (VEGF), such as bevacizumab (Avastin®), or targeting the receptor tyrosine kinase (RTK) VEGF receptor (VEGFR), such as sunitinib (Sutent®) and sorafenib (Nexavar®), have been approved for clinical use and are being widely employed. However, it has turned out that two modes of resistance in response to anti-angiogenic treatment hamper the efficacy of cancer therapy, leading to higher morbidity and mortality. These two modes of resistance are intrinsic non-responsiveness of tumours and evasive resistance (also called adaptive resistance, adaptive evasion or induced resistance). An overview about resistance to anti-angiogenic therapy is given for instance in Bergers & Hanahan, 2008.

Thus, efforts are needed to reduce or overcome resistance to established anti-angiogenic therapies.

### Summary of the invention

It is an object of the present invention to provide an agent that can be combined with an anti-angiogenic therapy, in particular a therapy with an RTK inhibitor such as sunitinib, in order to reduce or overcome resistance to the anti-angiogenic therapy.

Accordingly, the present invention provides a pharmaceutical composition comprising an apelin antagonist and/or an apelin receptor antagonist. The composition further comprises an angiogenesis inhibitor selected from the group of receptor tyrosine kinase (RTK) inhibitors. Herein, a combination of apelin antagonist (and/or apelin receptor antagonist) and RTK inhibitor is also called "the inventive combination".

The invention also relates to a method for prevention or treatment of cancer in a patient. The method comprises administration an effective amount of an apelin antagonist and/or apelin receptor antagonist to the patient. Furthermore, an angiogenesis inhibitor, preferably selected from the group of RTK inhibitors, is administered to the patient in an effective amount, in a composition together with the apelin antagonist and/or apelin receptor antagonist, in combination with the apelin antagonist and/or apelin receptor antagonist, before the apelin antagonist and/or apelin receptor antagonist is administered, or after the apelin antagonist and/or apelin receptor antagonist has been administered.

In the course of the present invention, it was surprisingly found that blockage of the apelin pathway overcame angiogenesis-inhibitor-induced resistance, in particular RTK-inhibitor induced resistance, in a cancer. The combination of apelin blockage with an RTK inhibition reduced vessel leakage, tumour malignancy and RTK-inhibitor-induced metastases in preclinical models of breast or lung cancer. Said combination therapy synergistically increased survival and reduced tumour burden (see Figs. 3 and 4). It was particularly surprising that the combination therapy did not further reduce tumour angiogenesis per se, despite achieving all the beneficial effects mentioned previously (see Fig. 5).

In further aspects of the invention, a set comprising the inventive combination in separate pharmaceutical compositions, and said apelin antagonist and/or apelin receptor antagonist for use in the prevention or treatment of cancer in a patient together with an angiogenesis inhibitor are provided. Furthermore, an angiogenesis inhibitor for use in the prevention or treatment of cancer in a patient together with said apelin antagonist and/or apelin receptor antagonist is provided.

### Detailed description of the invention

Apelin is an evolutionary conserved peptide that acts as the endogenous ligand for the G-protein-coupled APJ receptor (Tatemoto et al., 1998), also called apelin receptor. The apelin/APJ signalling pathway has been previously shown to play a role in angiogenesis (Kidoya and Takakura, 2012). Apelin is an angiogenic factor involved in vascular development (Cox et al., 2006; Kasai et al., 2008; Kälin et al., 2007; Saint-Geniez et al., 2002) and homoeostasis (del Toro et al., 2010; Kasai et al., 2004; Kidoya et al., 2008) and is also crucial for maintaining cardiac contractility (Kuba et al., 2007). Apelin has also been suggested to have a role in the aetiology of obesity (Rayalam et al., 2008). Apelin has been found to be up-regulated in certain mouse and human tumours (Berta et al., 2010; Sorli et al., 2007). Results from xenograft studies suggest that apelin over-expression in cancer cells affects the vascular component of the tumour by a paracrine action on endothelial cells of the host vessels (Berta et al., 2010; Sorli et al., 2007). Similar results have been reported following VEGF over-expression (Graeven et al., 2001), which plays a key role in tumour angiogenesis.

Despite all of these previous studies on the apelin signalling pathway, the inventive combination of the apelin antagonist and/or apelin receptor antagonist and the angiogenesis inhibitor (let alone the exceptional efficacy thereof, as shown herein) was not suggested before.

RTK inhibitors are well-known in the art as angiogenesis inhibitors, and they differ from VEGF antagonists such as the monoclonal antibody bevacizumab which form a distinct class of angiogenesis inhibitors (see e.g. Jeltsch et al, 2013). Unlike for instance the monoclonal antibody bevacizumab which binds to VEGF when it has been secreted from a cell and thereby inhibits binding of VEGF to its receptor on the surface of another cell, RTK inhibitors act inside the cell, specifically inhibiting the kinase activity of an RTK by binding to the kinase domain of the RTK. Typically, unlike antibodies, RTK inhibitors are small molecules (i.e. having a molecular weight below 800 Da) able to localise within cells (e.g. by diffusion), in particular ATP analogues with limited specificity. As an exemplary RTK, sunitinib is described in detail in Chow et al, 2007. For the purposes of the present invention, the RTK inhibitor can be any pharmaceutically acceptable, anti-angiogenic RTK inhibitor such as so-called "multikinase inhibitors". Suitable RTK inhibitors are also disclosed e.g. in WO 2001/060814 A2, WO 2002/059110 A1, WO 2003/068228 A1, US 6534524 B1 and US 7141581 B2, and in Bukowski et al., 2012 and Bhargava et al. 2011.

Preferably, the RTK inhibitor is a pharmaceutically acceptable RTK inhibiting the kinase activity of (in particular human) VEGFR-1, VEGFR-2, VEGFR-3 and/or VEGFR-4, and/or inhibiting the kinase activity of (in particular human) platelet-derived growth factor receptor (PDGFR)-alpha and/or PDGFR-beta.

In a preference, the RTK inhibitor has a half maximal inhibitory concentration (IC₅₀), in respect to one or more of (in particular human) VEGFR-1, VEGFR-2, VEGFR-3 and VEGFR-4, below 1000 nM, preferably below 500 nM, more preferably below 200 nM, even more preferably below 100 nM, especially below 50 nM or even below 10 nM in a cell-free assay.

In a further preference, the RTK inhibitor has an IC₅₀, in respect to one or more of (in particular human) PDGFR alpha and PDGFR beta, below 1000 nM, preferably below 500 nM, more preferably below 200 nM, even more preferably below 100 nM, especially below 50 nM or even below 10 nM in a cell-free assay.

Preferably, said cell-free assay is performed as disclosed in Sun et al., 1999, in particular in the sections "PDGF-R and EGF-R Kinase Assays" and "FGF-R1 and Flk-1/KDR Kinase Assays", optionally with modifications immediately apparent to the person skilled in the art (such as appropriately changing the receptor tyrosine kinase to be tested or the antibody binding thereto).

In a particular preferred embodiment of the present invention, the RTK inhibitor is selected from the group of sunitinib, sorafenib, pazopanib, axitinib, cabozantinib, regorafenib, vandetanib, tivozanib, cediranib, nintedanib and vatalanib. sunitinib, sorafenib, pazopanib and axitinib are especially preferred, among them sunitinib is particularly suitable for the present invention because it shows exceptionally strong therapeutic benefit in combination with apelin inhibition.

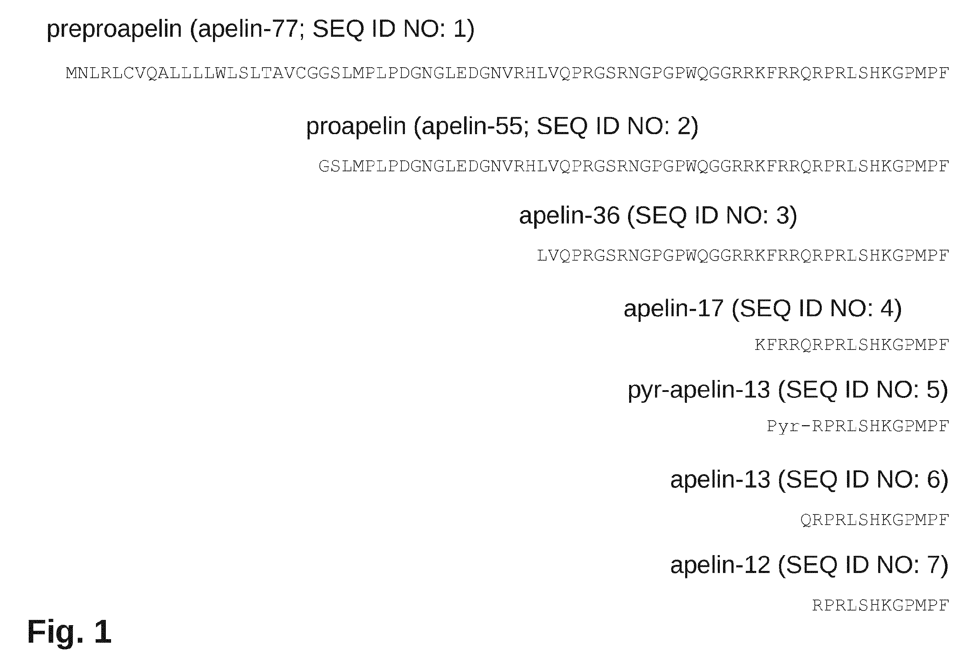

The human APLN gene (NCBI Entrez Gene ID: 8862) codes for an apelin preproprotein with 77 amino-acid residues ("apelin-77"; UniProt accession code Q9ULZ1, sequence version: 1) containing a signal peptide in its amino-terminal sequence thus directing apelin in the secretory pathway (see e.g. Chaves-Almagro et al, 2015; especially Fig. 1 thereof). Eventually the proprotein ("apelin-55") gives rise to several bioactive carboxy-terminal fragments including apelin-36, apelin-17 and apelin-13. The amino-terminal glutamine of apelin-13 can be post-translationally modified thus creating the pyroglutamate apelin-13 (pyr-apelin-13) which is more protected from exopeptidase degradation. An overview of human apelin variants is given in Fig. 1 (which is derived from Table 1 of Chapman et al., 2014).

The murine apelin preproprotein (UniProt accession code Q9R0R4, sequence version: 1) has the same length as the human homolog (i.e. murine apelin-77) and is highly similar, with an amino-acid identity of more than 80% to human apelin-77. The functionally especially relevant carboxy-terminal residues, in particular apelin-17 and apelin-13, are 100% identical between the two species, making the mouse an exceptionally suitable model system for apelin-related therapy.

In the context of the present invention, the apelin antagonist can be any compound that specifically binds apelin and reduces or inhibits binding of apelin to the apelin receptor, e.g. by steric hindrance, or by reducing apelin stability e.g. in an extracellular body fluid such as blood serum. Preferably, the apelin antagonist is specific for one or more of apelin-77, apelin-55, apelin-36, apelin-17, pyr-apelin-13, apelin-13 and apelin-12 as defined in Fig. 1.

In a particularly preferred embodiment, the apelin antagonist is an anti-apelin antibody, in particular a monoclonal anti-apelin antibody. Preferably, the, preferably monoclonal, antibody is specific for one or more of apelin-77, apelin-55, apelin-36, apelin-17, pyr-apelin-13, apelin-13 and apelin-12 as defined in Fig. 1, especially one or more of apelin-36, apelin-17, pyr-apelin-13 and apelin-13. Suitable epitopes can be predicted by methods known in the art, e.g. by the method of Larsen et al. 2006, which is also conveniently available for the skilled person as an online tool e.g. on http://tools.iedb.org/bcell/ and also provides other epitope prediction methods). In particular, the, preferably monoclonal, anti-apelin antibody is specific for one or more of the following peptide epitopes: 5, 6, 7, 8, 9, 10, 11, 12, 13, 14, 15, 16, 17, 18, 19 or 20 consecutive amino acids selected from the amino acid sequence VQPRGSRNGPGPWQG-GRRKFR (SEQ ID NO: 8), preferably 5, 6, 7, 8, 9, 10, 11 or 12 consecutive amino acids selected from the amino acid sequence RGSRNGPGPWQG (SEQ ID NO: 9), especially 5, 6 or 7 consecutive amino acids selected from the amino acid sequence RNGPGPW (SEQ ID NO: 10); or 5, 6, 7, 8, 9, 10 or 11 consecutive amino acids selected from the amino acid sequence PRLSHKGPMPF (SEQ ID NO: 11), preferably 5, 6 or 7 consecutive amino acids selected from the amino acid sequence PRLSHKG (SEQ ID NO: 12) or 5, 6, 7 or 8 consecutive amino acids selected from the amino acid sequence LSHKGPMP (SEQ ID NO: 13).

Many anti-apelin antibodies suitable for the present invention are known in the prior art. For instance, the WO 2013/012855 A1 discloses a monoclonal anti-apelin antibody specific for the epitope PRLSHKG (SEQ ID NO: 12; present in all forms of apelin shown in Fig. 1) that is particularly suitable for the present invention. Another suitable monoclonal antibody, specific for at least the apelin-13 form, is disclosed e.g. in Kawamata et al., 2001. Suitable polyclonal antibodies raised against apelin-17 are disclosed e.g. in Reaux et al., 2002. Suitable anti-apelin antibodies are for instance also commercially available from Santa Cruz Biotechnology Inc. (Dallas, TX, USA) or Abcam plc (Cambridge, UK).

Both expression and purification of monoclonal antibodies is well-known in the art. See for instance Birch & Racher, 2006, for an overview of large-scale antibody production (expression and purification). Techniques also comprise conventional monoclonal antibody methodology, for example the standard somatic cell hybridization technique of Köhler and Milstein. Other techniques for producing monoclonal antibodies can also be employed such as viral or oncogenic transformation of B lymphocytes.

The monoclonal anti-apelin antibody is preferably humanised. Methods to obtain humanised antibodies are well known in the art. One method is to insert the variable regions of the antibody (e.g. the variable regions of the monoclonal antibody disclosed in the WO 2013/012855 A1, especially Fig. 1 thereof) into a human antibody scaffold. See e.g. Almagro & Franson, 2008, for an overview of humanisation techniques. For facilitated humanisation, monoclonal antibodies can also be raised in genetically modified mice, such as the VELOCIMMUNE® mouse; see e.g. the US patents no. 6,596,541 and US 7,105,348.

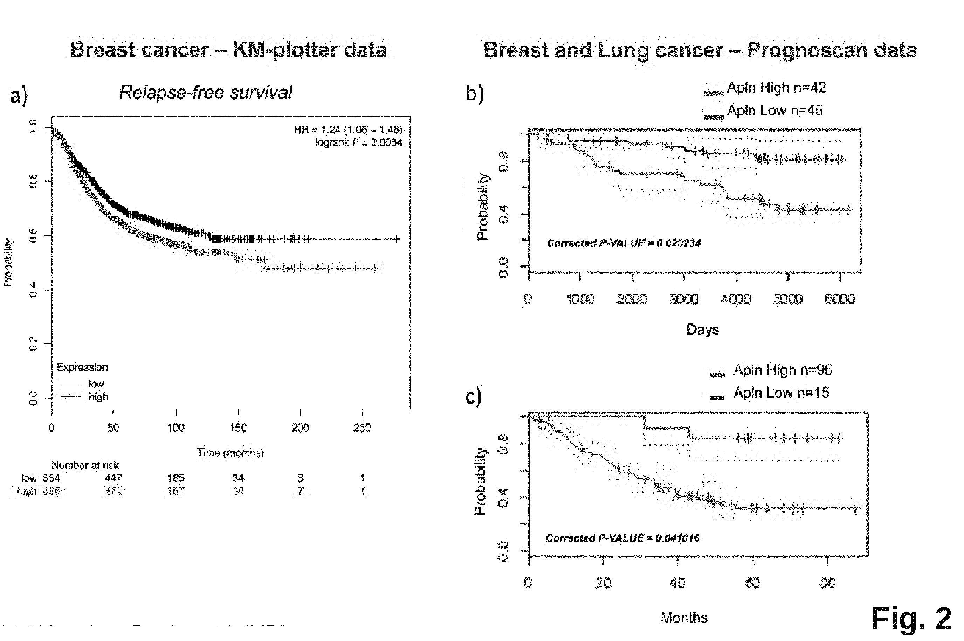

The human APLNR gene (NCBI Entrez Gene ID: 187) codes for the human apelin receptor (cf. Chaves-Almagro et al, 2015; especially Fig. 2 thereof). The gene encodes a protein of 380 amino acids (UniProt accession code P35414, sequence version: 1) which belongs to the class A (rhodopsin-like receptor) G protein-coupled receptor (GPCR) family. Glu20 and Asp23 in the extracellular N-terminal tail were first identified as crucial residues for binding of its endogenous ligand apelin. In addition, it was recently established that Asp94, Glu174 and Asp284 are also involved in apelin binding.

In the context of the present invention, the apelin receptor antagonist can be any compound that specifically binds apelin receptor and reduces or inhibits binding of apelin to the apelin receptor, e.g. by steric hindrance, or by reducing apelin receptor stability. Preferably, the apelin receptor antagonist is specific for human apelin receptor.

In a further preferred embodiment of the present invention, the apelin receptor antagonist is an anti-apelin-receptor antibody, in particular a monoclonal anti-apelin-receptor antibody. Suitable epitopes can be predicted by methods known in the art, e.g. by the method of Larsen et al. 2006, which is also conveniently available for the skilled person as an online tool e.g. on http://tools.iedb.org/bcell/ and also provides other epitope prediction methods). Preferably, the epitope includes at least one of the amino acid sites of the receptor known to be involved in apelin binding, in particular selected from Glu20, Asp23, Asp94, Glu174 and Asp284 (according to the numbering of UniProt accession code P35414, sequence version: 1). In particular, the, preferably monoclonal, anti-apelin-receptor antibody is specific for one or more of the following peptide epitopes: 5, 6, 7, 8, 9, 10, 11, 12, 13, 14, 15, 16, 17, 18, 19 or 20 consecutive amino acids selected from the amino acid sequence MEEGGDFDNYY-GADNQSECEYTDWKS (SEQ ID NO: 15), preferably 5, 6, 7, 8, 9, 10, 11, 12, 13, 14, 15, 16, 17, 18, 19 or 20 consecutive amino acids selected from the amino acid sequence GGDFDNYYGADNQSECEYTD (SEQ ID NO: 16), more preferably 5, 6, 7, 8, 9, 10, 11, 12, 13, 14 or 15 consecutive amino acids selected from the amino acid sequence NYYGADNQSECEYTD (SEQ ID NO: 17), even more preferably 5, 6, 7, 8, 9 or 10 consecutive amino acids selected from the amino acid sequence DNQSECEYTD (SEQ ID NO: 18), especially 5, 6, or 7 consecutive amino acids selected from the amino acid sequence SE-CEYTD (SEQ ID NO: 19); or 5, 6, 7 or 8 consecutive amino acids selected from the amino acid sequence GDLENTTK (SEQ ID NO: 20), preferably 5 or 6 consecutive amino acids selected from the amino acid sequence DLENTT (SEQ ID NO: 21).

Many anti-apelin-receptor antibodies suitable for the present invention are known in the prior art. Anti-apelin-receptor antibodies are for instance disclosed in WO 1999/033976 A1. Also suitable for the present invention is the monoclonal anti-apelin-receptor antibody MAB856 disclosed in Puffer et al., 2000. This antibody is commercially available as MAB856 from R&D Systems, Inc., Minneapolis, MN, USA. Especially suitable for the present invention is an antibody, antibody-fusion protein or antigen-binding fragment as claimed in any one of claims 1-14 of the WO 2015/077491 A1, especially when the sequence specified in the claims (cf. Table 1 of WO 2015/077491 A1) is drawn to any one of H1M9207N, H2aM9209N, H2aM9222N, H2aM9227N, H2aM9228N, H2aM9230N and H2aM9232N, in particular to H2aM9232N.

The monoclonal anti-apelin-receptor antibody is preferably humanised.

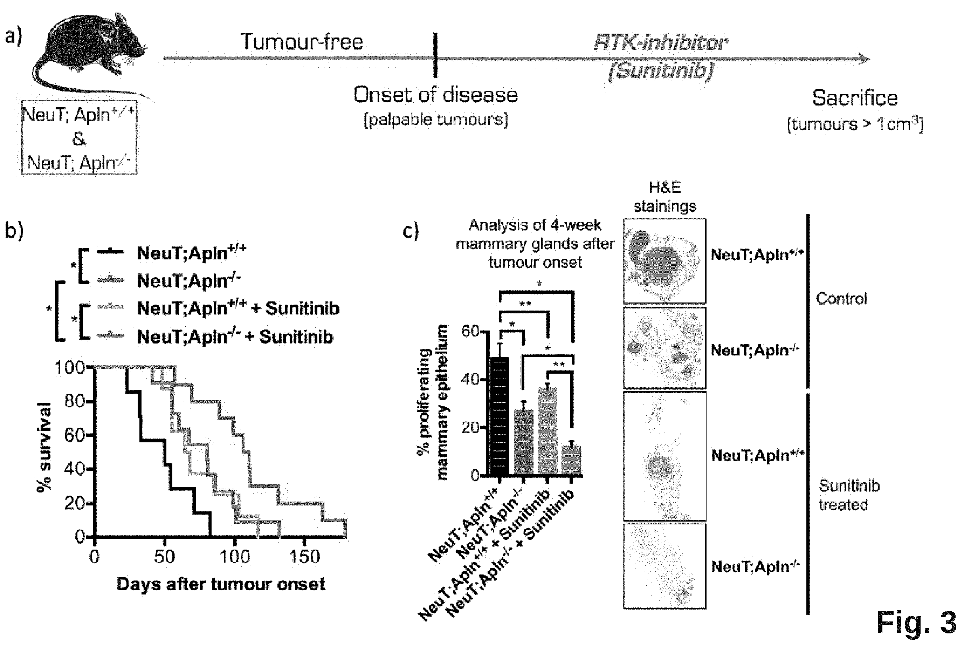

In a further preferred embodiment of the present invention, the apelin receptor antagonist is an apelin analogue. The apelin analogue is structurally similar to apelin and inhibits activation of the apelin receptor by apelin. Preferably the inhibition is by competing with apelin for the apelin binding site on the receptor. Advantageously, when applied in the presence of apelin, preferably apelin-36, apelin-17, pyr-apelin-13, apelin-13 or apelin-12, especially apelin-36 or apelin-13, at the same concentration (molar or mass), the analogue decreases apelin-induced apelin receptor activation by a factor of at least 3, preferably of at least 5, more preferably of at least 10, especially of at least 50. Apelin receptor activation can be measured in vitro or in vivo, as e.g. disclosed in Chaves-Almagro et al., 2015, especially p. 152, section 2.5; in particular by measuring decrease of mean arterial blood pressure in anaesthetized rats (see Lee et al, 2005; in particular Fig. 3B and section "Blood pressure and heart rate bioassay") or according to the cyclic AMP assay disclosed in Example 5 of the WO 2015/077491 A1. It is preferred that the apelin analogue is a peptide. In a particular preference, the apelin analogue is the peptide apelin F13A (QRPRLSHKGPMPA; SEQ ID NO: 14). Apelin F13A was characterized e.g. in Lee et al, 2005. The apelin analogue can also be the peptide ALX40-4C (N-α-acetyl-nona-D-arginine amide; SEQ ID NO: 22) which was characterized e.g. in Zhou et al, 2003, although higher sequence identity of the antagonist to apelin (e.g. apelin-36 or apelin-13) is preferred to increase specificity for the apelin receptor (of course, while still retaining its antagonistic property in regard to the apelin receptor, e.g. by appropriate point mutations such as in the case of apelin F13A), e.g. at least 50% sequence identity, preferably at least 60% sequence identity, more preferably at least 70% sequence identity, even more preferably at least 80% sequence identity, in particular at least 90% sequence identity.

Further apelin receptor antagonists suitable for the present invention are also known in the art: For instance, Macaluso et al., 2011, discloses cyclo(1-6)CRPRLC-KH-cyclo(9-14)CRPRLC (SEQ ID NO: 23) as a competitive antagonist of apelin receptor. The apelin receptor antagonist 4-oxo-6-((pyrimidin-2-ylthio)methyl)-*4H*-pyran-3-yl 4-nitrobenzoate (ML221) is disclosed e.g. in Maloney et al, 2012. Further suitable apelin receptor antagonists are disclosed e.g. in the US 2014/0005181 A1, the WO 2010/053545 A2 and the WO 2014/044738 A1. Further apelin antagonists or apelin receptor antagonists are disclosed in WO 2015/140296 A2.

If necessitated e.g. due to different modes of administration of the components, the inventive combination can be provided as a set or kit-of-parts. Therefore, the present invention is drawn, in a further aspect, to a set comprising a first pharmaceutical composition and a second pharmaceutical composition. The first pharmaceutical composition comprises the apelin antagonist and/or the apelin receptor antagonist. The second pharmaceutical composition comprises the angiogenesis inhibitor selected from the group of RTK inhibitors. The set is advantageously provided in the same packaging, wherein the first composition is in a first container (e.g. in liquid form) and the second composition is in a second container (e.g. in tablet form).

In a further aspect, the invention relates to an apelin antagonist and/or apelin receptor antagonist, or an angiogenesis inhibitor, for use in the prevention or treatment of cancer in a patient. The apelin antagonist and/or apelin receptor antagonist is administered to the patient. An angiogenesis inhibitor, preferably selected from the group of RTK inhibitors, is administered to the patient, in a composition together with the apelin antagonist and/or apelin receptor antagonist, in combination with the apelin antagonist and/or apelin receptor antagonist, before the apelin antagonist and/or apelin receptor antagonist is administered, or after the apelin antagonist and/or apelin receptor antagonist has been administered. This may alternatively be phrased as: an apelin antagonist and/or apelin receptor antagonist, being administered to the patient, in a composition together with the angiogenesis inhibitor, in combination with the angiogenesis inhibitor, before the angiogenesis inhibitor is administered, or after the angiogenesis inhibitor has been administered. Preferably, the inventive treatment is for increasing drug delivery (e.g. of a further chemotherapeutic agent), reducing tumour growth (either an absolute reduction of mass, or a reduction of mass increase per time interval), preventing malignancy and/or preventing metastasis (in particular metastasis induced by anti-angiogenic treatment, e.g. with an RTK inhibitor such as sunitinib). Typically, the tumour to be treated is a solid tumour.

In a further aspect, the invention relates to an apelin antagonist and/or apelin receptor antagonist (or an angiogenesis inhibitor) for use in the prevention or treatment of metastasis in a cancer patient, wherein the patient has undergone or is undergoing cancer treatment with an angiogenesis inhibitor (or an apelin antagonist and/or apelin receptor antagonist). The same forms of administration as described above can be used.

In a further aspect, the invention relates to an apelin antagonist and/or apelin receptor antagonist for use in the treatment of a cancer patient who has resistance or is at risk of developing resistance to anti-angiogenic therapy. This aspect also relates to an angiogenesis inhibitor for use in the treatment of a cancer patient who has resistance or is at risk of developing resistance to anti-angiogenic therapy, wherein the angiogenesis inhibitor is administered to the patient, and wherein an apelin antagonist and/or apelin receptor antagonist, is administered to the patient, in a composition together with the angiogenesis inhibitor, in combination with the angiogenesis inhibitor, before the angiogenesis inhibitor is administered, or after the angiogenesis inhibitor has been administered.

Resistance to anti-angiogenic therapy can be characterized by non-responsiveness of the cancer patient's tumour or relapse after an initial response, as described for instance in Bergers et al., 2008 and Jayson et al, 2016. Preferably, non-responsiveness is defined as fulfilling the criteria for "stable disease" or "progressive disease" (in particular the latter) according to the version 1.1 of RECIST guidelines (Eisenhauer et al., 2009, in particular item 4.3.1 thereof) after having been (e.g. at least 2 weeks, preferably at least 4 weeks, more preferably at least 8 weeks, even more preferably at least 12 weeks, especially at least 16 weeks or even at least 24 weeks) on anti-angiogenic treatment. Preferably, relapse is defined as fulfilling the criteria for "progressive disease" according to version 1.1 of the RECIST guidelines (Eisenhauer et al., 2009, in particular item 4.3.1 thereof), after the criteria for "complete response" or "partial response" (especially the former) had initially been fulfilled according to said guidelines (see Eisenhauer et al., 2009, in particular item 4.3.1 thereof) upon (e.g. at least 2 weeks, preferably at least 4 weeks, more preferably at least 8 weeks, even more preferably at least 12 weeks, especially at least 16 weeks or even at least 24 weeks) anti-angiogenic treatment.

In particular, a cancer patient is at risk of developing resistance to anti-angiogenic therapy if the tumour/cancer is Kras-positive (as defined above), over-expresses granulocyte-colony stimulating factor (G-CSF) (compared to an appropriate control), and/or over-expresses P-Rex1 (compared to an appropriate control), see e.g. Phan et al., 2013 and Goel et al, 2016. Alternatively, or in addition thereto, a cancer patient is, in particular, at risk of developing resistance to anti-angiogenic therapy if obese (i.e. the patient has a body mass index of at least 30 kg/m², especially at least 35 kg/m²), see e.g. Incio et al, 2015.

The detailed description herein relates to all aspects of the invention unless explicitly excluded.

In the context of the above-mentioned three aspects, the angiogenesis inhibitor can also be a VEGF antagonist such as the monoclonal antibody bevacizumab. However, using an RTK inhibitor (as explained in detail above, an RTK inhibitor is not a VEGF antagonist), such as sunitinib, is preferred as it is particularly effective. The VEGF antagonist can be any compound that specifically binds VEGF and reduces or inhibits binding of VEGF to the VEGF receptor, e.g. by steric hindrance, or by reducing VEGF stability e.g. in an extracellular body fluid such as blood serum. Suitable are for instance anti-VEGF antibodies (such as bevacizumab and ranibizumab) or VEGF traps (such as aflibercept). In regard to the apelin antagonist and/or apelin receptor antagonist, the definitions and preferred embodiments laid out above also apply to the present context.

In an embodiment, the cancer to be treated or prevented is selected from breast cancer, lung cancer, ovarian cancer, cervical cancer, hepatocellular carcinoma, renal cell carcinoma (RCC), especially clear-cell RCC, thyroid cancer, neuroendocrine cancer, gastro-oesophageal cancer, colorectal cancer, glioma, prostate cancer and pancreatic cancer. Typically, the cancer can be of epithelial origin (i.e. carcinoma or epithelial cancer).

In an embodiment, especially if the cancer is breast cancer and/or especially in connection to the inventive prevention of cancer, the patient has a loss-of-function mutation or deletion of one or more of the genes BRCA1, BRCA2, BRCA3 and RAD51D.

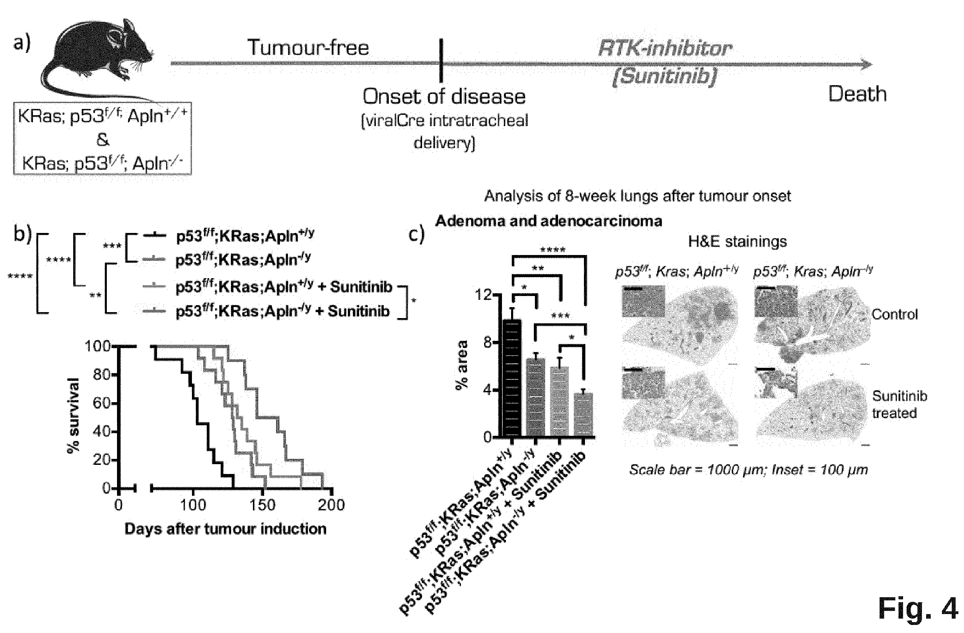

The present invention is particularly effective in breast cancer and lung cancer, as shown in particular in Fig. 3 and 4. Accordingly, in further, especially preferred embodiment the cancer is selected from breast cancer and lung cancer (especially non-small cell lung cancer). Typically, the cancer is of epithelial origin (i.e. carcinoma or epithelial cancer).

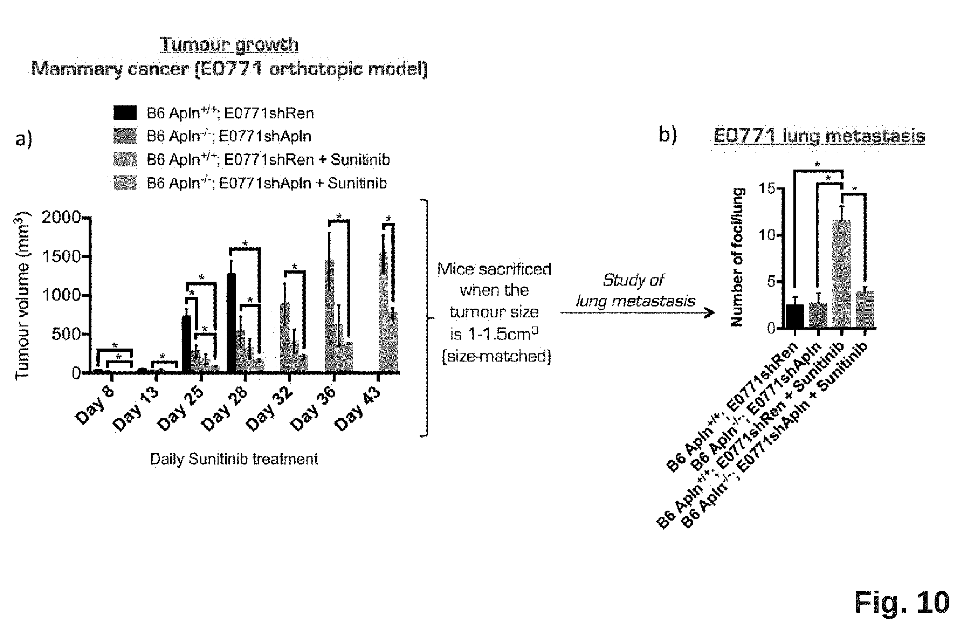

In the course of the present invention, it was found that the present invention is particularly effective in cancers over-expressing certain oncogenes or lacking certain tumour suppressor genes, i.e. cancers that are HER-2-positive (see Fig. 3b; Neu is the rodent homolog of HER-2, with NeuT being a particularly oncogenic form thereof), p53-negative (see Fig. 4b), KRas-positive (see also Fig. 4b), estrogen-receptor-(ER)-positive (see also Fig. 10, the human breast cancer cell line E0771 is ER-positive), or combinations thereof. Therefore, in a further preference, the cancer is HER-2-positive (i.e. over-expressing HER-2 compared to healthy control tissue), p53-negative (i.e. not expressing p53 or under-expressing p53 compared to healthy control tissue or having mutated p53), Kras-positive (i.e. over-expressing KRas compared to healthy control tissue or having mutated KRas), ER-positive (i.e. over-expressing ER compared to healthy control tissue) or combinations thereof. Preferably, the cancer is HER-2-positive, ER-positive and/or p53-negative breast cancer (or HER-2-negative, p53-negative and/or ER-positive breast cancer); or HER-2-positive and/or p53-negative and/or KRas-positive lung cancer (especially non-small cell lung cancer). The oncogene (or tumour suppressor) status of a cancer can be tested e.g. by immunohistochemistry (IHC) or fluorescence in-situ hybridisation; testing for oncogene (or tumour suppressor) status of a cancer is well-established clinical practice.

In a particularly preferred embodiment, status of HER-2, ER, KRas and p53 is defined in the following way: The cancer is HER2-positive when an intense and uniform complete HER-2 membrane staining is observed in more than 10% of the invasive tumour cells in an IHC assay, and/or, in an in-situ hybridisation assay such as FISH or bright field ISH, when the dual-probe HER2/CEP17 ratio is at least 2.0 or the average HER2 copy number is at least 6.0 signals per cell; in particular as disclosed in Rakha et al., 2015, especially Fig. 1 thereof. The cancer is ER-positive when it has an Allred score of at least 3 (the Allred scoring system is disclosed e.g. in Harvey et al., 1999). Guidelines for immunohistochemical testing of ER are for instance given in Hammond et al., 2010. The cancer is Kras-positive when it harbours a mutation in the KRas gene or protein (see e.g. van Cutsem et al., 2009 for testing methods), preferably leading to a KRas protein with higher GTPase activity than wild-type KRas. The cancer is p53-negative when it does not have p53 gene or protein or harbours a mutation in the p53 gene or protein interfering with wild-type p53 function.

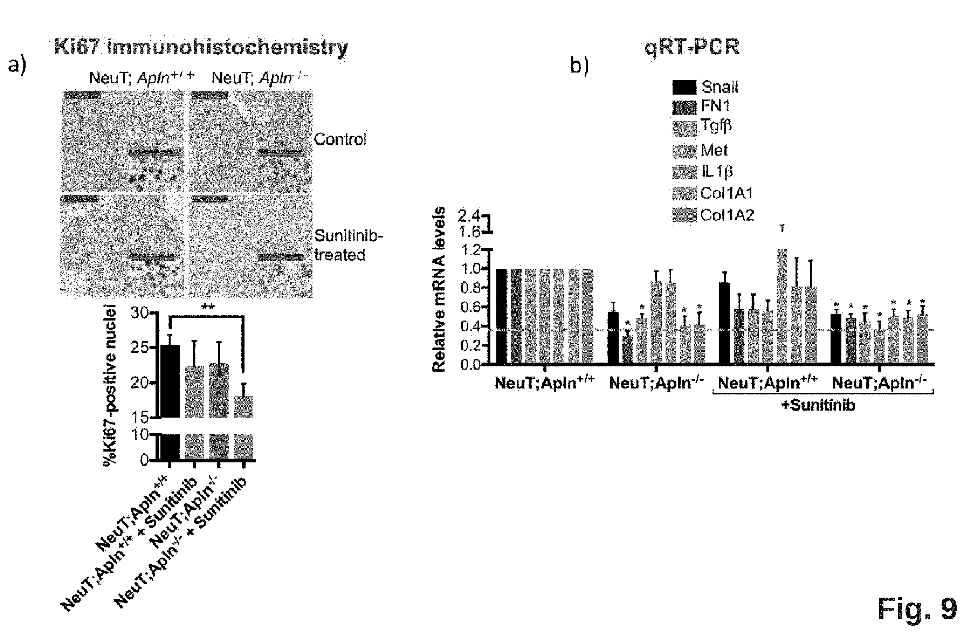

The present invention has been shown to effectively reduce expression of epithelial-mesenchymal transition (EMT) markers and invasiveness-related genes (see Fig. 9b). As such, in a preferred embodiment of the present invention the cancer over-expresses EMT markers and/or over-expresses invasiveness-related genes (compared to an appropriate control, e.g. healthy adjoining tissue). Suitable EMT markers are known from e.g. Lamouille et al. 2014, in particular Table 1. Preferably, the EMT marker used is selected from the group of Snail (especially SNAIL1 and SNAIL2), fibronectin (especially fibronectin 1), and TGF-beta. Suitable invasiveness-related genes are known from e.g. Ho-Yen et al., 2015, Martin et al., 2013, Gherardi et al, 2012, Apte et al., 2006, and Kalluri, et al., 2006. Preferably, the invasiveness-related gene is selected from the group of Met, IL1b, COL1A1 and COL1A2.

The present invention is extraordinarily effective in cancers that over-express apelin or apelin receptor. Thus, in a preferred embodiment of the present invention the cancer over-expresses apelin and/or apelin receptor (compared to an appropriate control, e.g. healthy adjoining tissue).

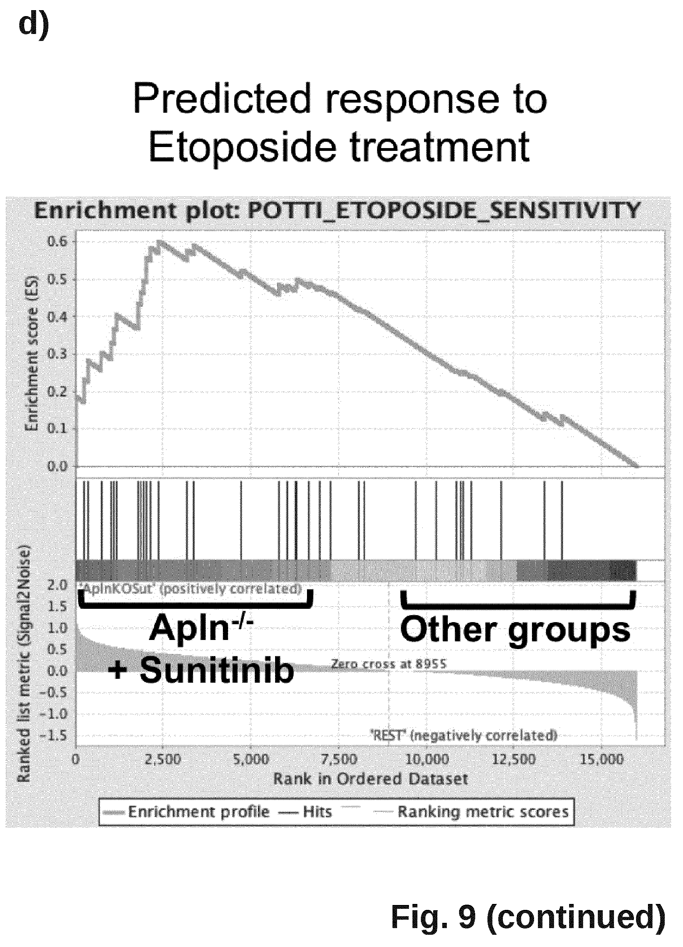

A further preferred embodiment of the present invention relates to the apelin antagonist and/or apelin receptor antagonist, or the angiogenesis inhibitor, for use, wherein a further chemotherapeutic agent is administered to the patient, in a composition together with the apelin antagonist and/or apelin receptor antagonist, or the angiogenesis inhibitor, in combination with the apelin antagonist and/or apelin receptor antagonist, or the angiogenesis inhibitor, before the apelin antagonist and/or apelin receptor antagonist, or the angiogenesis inhibitor, is administered, or after the apelin antagonist and/or apelin receptor antagonist, or the angiogenesis inhibitor, has been administered. The further chemotherapeutic agent is selected from the group of alkylating agents, platin-based chemotherapeutic agents, antimetabolites, anti-microtubule agents, topoisomerase inhibitors and cytotoxic antibiotics. Preferably, the further chemotherapeutic agent is selected from the group of 5-fluorouracil, cisplatin, carboplatin, cyclophosphamide, doxorubicin, epirubicin, gemcitabine, taxanes such as paclitaxel or docetaxel, methotrexate, pemetrexed, vinorelbine and etoposide. Topoisomerase inhibitors (in particular topoisomerase II inhibitors such as etoposide) are especially preferred, as cancers treated by the inventive combination are particularly sensitive to this additional treatment (see Fig. 9d). Finally, in the context of the present invention, apelin antagonists, apelin receptor antagonists, angiogenesis inhibitors, RTK inhibitors and Src tyrosine kinase inhibitors, especially as disclosed herein, are not the be understood as "further chemotherapeutic agents".

In addition to the previous embodiment, or alternatively thereto, in a further embodiment, radiotherapy is administered to the patient, in combination with the apelin antagonist and/or apelin receptor antagonist, or the angiogenesis inhibitor, before the apelin antagonist and/or apelin receptor antagonist, or the angiogenesis inhibitor, is administered, or after the apelin antagonist and/or apelin receptor antagonist, or the angiogenesis inhibitor, has been administered.

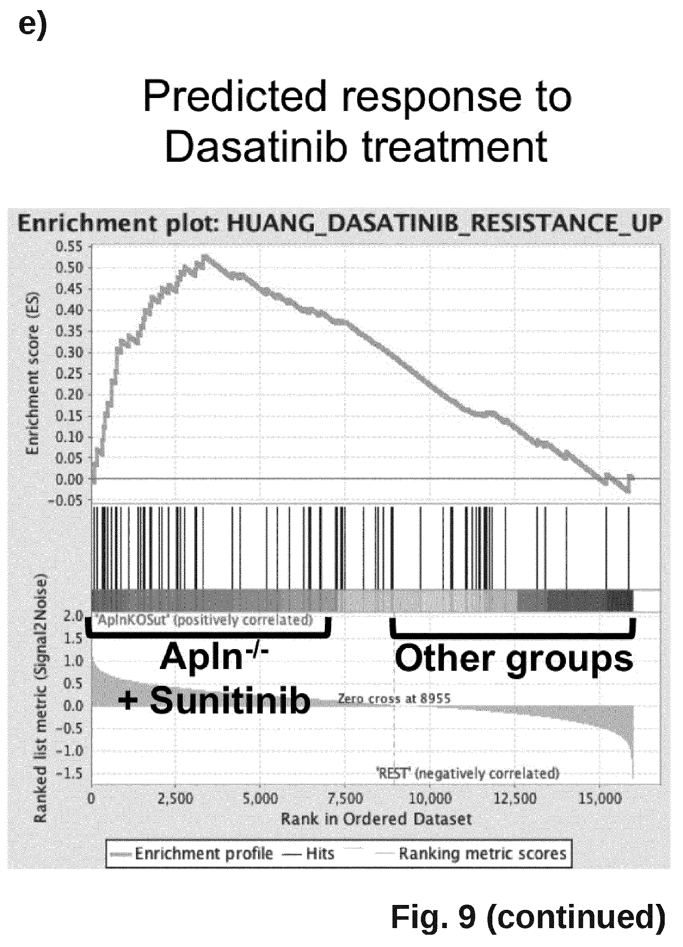

The tyrosine kinase Src is a cytoplasmic tyrosine kinase encoded by the proto-oncogene SRC. Evidently, Src tyrosine kinase is not an RTK. In the course of the present invention it was found that cancers treated with the inventive combination are particularly sensitive to Src tyrosine kinase inhibitor treatment (see Fig. 9e). Therefore, a further preferred embodiment of the present invention relates to the apelin antagonist and/or apelin receptor antagonist, or the angiogenesis inhibitor, for use, wherein a Src tyrosine kinase inhibitor is administered to the patient, in a composition together with the apelin antagonist and/or apelin receptor antagonist, or the angiogenesis inhibitor, in combination with the apelin antagonist and/or apelin receptor antagonist, or the angiogenesis inhibitor, before the apelin antagonist and/or apelin receptor antagonist, or the angiogenesis inhibitor, is administered, or after the apelin antagonist and/or apelin receptor antagonist, or the angiogenesis inhibitor, has been administered.

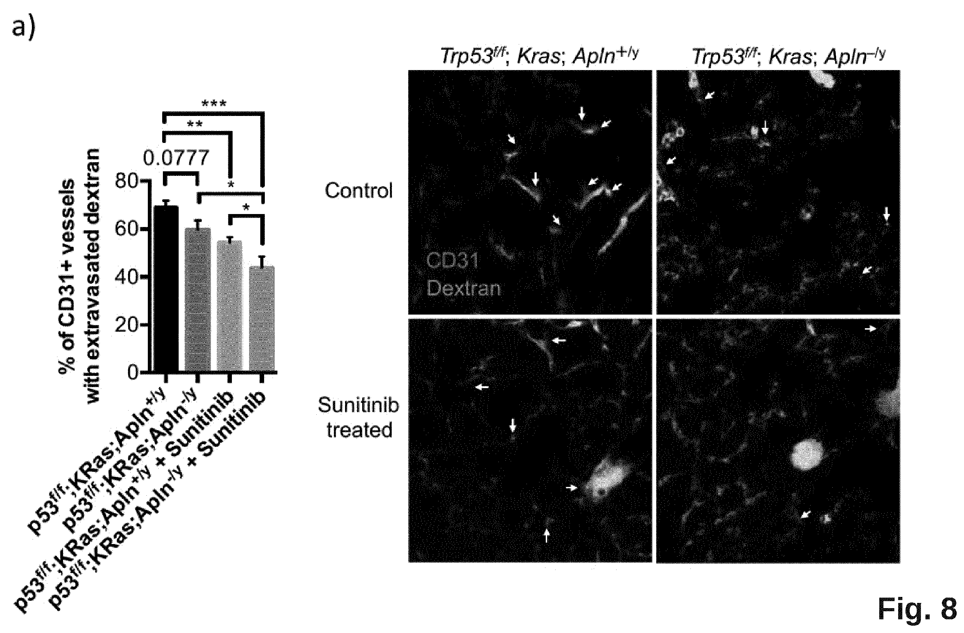

Typically, Src tyrosine kinase inhibitors are small molecules (i.e. having a molecular weight below 800 Da) able to localise within cells (e.g. by diffusion), in particular ATP analogues with limited specificity. Suitable Src tyrosine kinase inhibitors are for instance described in Rososki 2015 (in particular Table 4 and Fig. 8 thereof) and throughout Schenone, et al., 2011. In the context of the present invention, the Src tyrosine kinase inhibitor is any pharmaceutically acceptable compound inhibiting the kinase activity of the protein Src (in particular human Src), preferably by binding to Src.

In a preference, the Src tyrosine kinase inhibitor has a half maximal inhibitory concentration (IC₅₀) in respect to (in particular human) Src below 1000 nM, preferably below 500 nM, more preferably below 200 nM, even more preferably below 100 nM, especially below 50 nM or even below 10 nM in a cell-free assay.

Preferably, said cell-free assay is performed as disclosed in WO 2007/014707 A1, in particular on p. 16, line 17 to p. 17, line 20, or as disclosed in WO 02/092573 A2, in particular in the section "Src Inhibition Assays" beginning on p. 47; optionally with modifications immediately apparent to the person skilled in the art.

The Src tyrosine kinase inhibitor is preferably selected from bosutinib, dasatinib, ponatinib, vandetanib, saracatinib and AZD0424, especially dasatinib.

It was found in the course of the present invention that breast cancer patients and lung cancer patients with higher apelin levels have a worse disease outcome (see for instance Fig. 2). Accordingly, measuring apelin levels facilitates selection of a patient population that will profit extraordinarily from the inventive treatment (especially in breast cancer or lung cancer). Thus, in a further embodiment of the present invention, the patient has an apelin level, preferably a plasma or serum apelin level, higher than an appropriate control (such as a healthy age-matched individual or an average of a group of healthy individuals). Alternatively, or in addition thereto, the cancer (as measured e.g. in a biopsy of the primary tumour) has an apelin or apelin receptor level higher than an appropriate control (such as healthy adjoining tissue). Advantageously, the apelin (or apelin receptor) level is measured after the patient has undergone cancer treatment with an angiogenesis inhibitor, preferably selected from the group of RTK inhibitors, especially sunitinib. This can serve to predict anti-angiogenic resistance. In particular, said measurement occurs before treatment with the apelin antagonist or apelin receptor antagonist is started. Preferably, only if the apelin (receptor) level is above a certain threshold the apelin antagonist or apelin receptor antagonist is administered. The threshold can be at 110% of the control apelin (receptor) level, preferably at 120%, more preferably at 130%, even more preferably at 140%, especially at 150% or even at 200% or 300%.

Measuring the apelin level, especially in plasma or serum of a patient, is known in the art, see for instance Alexiadou et al, 2012, Angelova et al., 2014, or Zhang et al., 2009. ELISA kits for measuring apelin levels are commercially available e.g. from Phoenix Pharmaceuticals, Inc, Burlingame, USA. Typically, an aggregate apelin level of apelin-36, apelin-17, apelin-13, pyr-apelin-13 and apelin-12 is measured. However, in an embodiment, the apelin level measured can be selected from one or more of the level of apelin-36, apelin-17, apelin-13, pyr-apelin-13 and apelin-12, preferably from apelin-17, apelin-13, pyr-apelin-13 and apelin-12, especially from apelin-13 and pyr-apelin-13. The apelin receptor level can be measured for instance with the anti-apelin-receptor antibodies disclosed herein in an ELISA or a immunohistochemistry assay performed e.g. of tumour cells. The skilled person knows how to select an appropriate control or control group in regard to the apelin or apelin receptor level. Preferably, for plasma or serum apelin levels, the control group is (at least essentially) matched for one or more of age, body mass index and sex. Also preferably, the blood sample is taken at a fasting state, e.g. after 12 hours of fasting, for both the patient and the control. In a further preferred embodiment, the control is the apelin level of the patient, preferably in plasma or serum obtained from the patient, or the apelin receptor level of the (primary) tumour of the patient (e.g. of a tumour sample obtained by biopsy), before the patient has undergone cancer treatment with the angiogenesis inhibitor, preferably selected from the group of RTK inhibitors, especially sunitinib. Preferably, only if the apelin (receptor) level is above a certain threshold (e.g. as disclosed in the previous paragraph) the apelin antagonist and/or apelin receptor antagonist is administered.

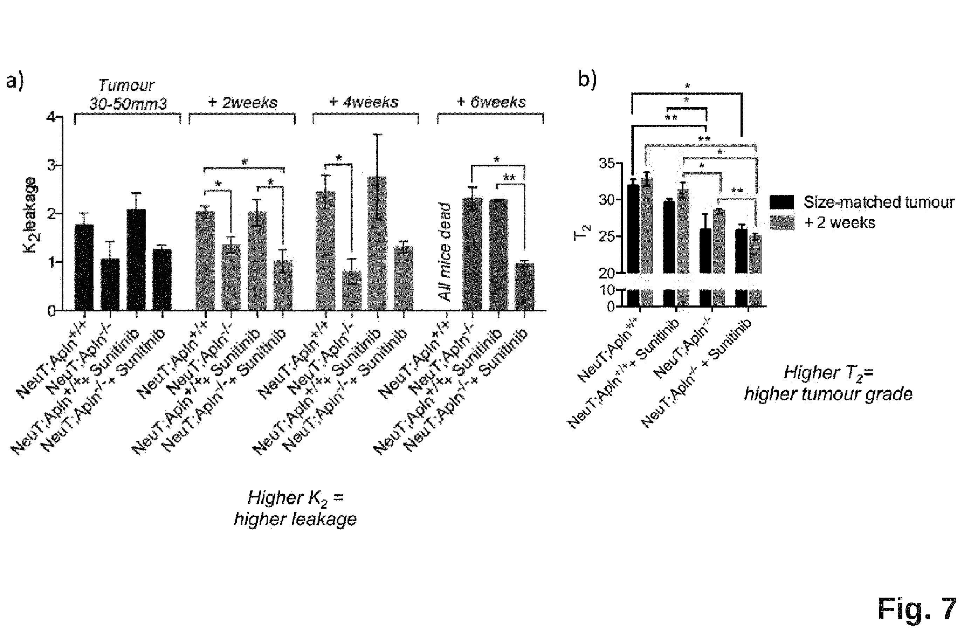

Blood vessels of tumours are typically leaky and poorly organized. In particular, this may hamper delivery of cytotoxic drugs to a cancer patient's tumour. Therefore, in a preference, tumour blood vessel normalisation (in other words: reduction of tumour blood vessel leakiness and/or reduction of interstitial pressure) in the patient is achieved by the inventive preventive therapy or treatment; or, preferably, the present invention is for tumour blood vessel normalisation (again, in other words: reduction of tumour blood vessel leakiness and/or reduction of interstitial pressure). This beneficial effect of the inventive treatment is for instance shown in Figs. 7 and 8. Blood vessel normalisation can be measured by methods known in the art, such as magnetic resonance imaging (MRI) (cf. Fig. 7) and fluorescence microscopy of dextran extravasation (cf. Fig. 8).

In a further aspect, the present invention provides a pharmaceutical composition comprising an apelin antagonist (as disclosed above) and/or apelin receptor antagonist (as disclosed above) and a further chemotherapeutic agent. This composition is preferably prepared for parenteral, especially intravenous, administration. Alternatively, the components can also be provided as a set or kit-of-parts, i.e. a set comprising a first pharmaceutical composition comprising an apelin antagonist and/or apelin receptor antagonist (as disclosed above) and a second pharmaceutical composition comprising a further chemotherapeutic agent. The further chemotherapeutic agent is selected from the group of alkylating agents, platin-based chemotherapeutic agents, antimetabolites, anti-microtubule agents, topoisomerase inhibitors and cytotoxic antibiotics. Preferably, the further chemotherapeutic agent is selected from the group of 5-fluorouracil, cisplatin, carboplatin, cyclophosphamide, doxorubicin, epirubicin, gemcitabine, taxanes such as paclitaxel or docetaxel, methotrexate, pemetrexed, vinorelbine and etoposide. Topoisomerase inhibitors (in particular topoisomerase II inhibitors such as etoposide) are especially preferred. Finally, in the context of the present invention, apelin antagonists, apelin receptor antagonists, angiogenesis inhibitors, RTK inhibitors and Src tyrosine kinase inhibitors, especially as disclosed herein, are not the be understood as "further chemotherapeutic agents". The composition or set can be used for any one of the therapeutic uses disclosed herein, such as treatment of breast cancer or lung cancer, e.g. in a cancer patient with resistance or at risk of developing resistance to an anti-angiogenic therapy.

In a still further aspect, the present invention relates to the further chemotherapeutic agent as defined above for use in treatment of a cancer, or in prevention or treatment of cancer metastasis, in a cancer patient. In this aspect, the patient has undergone or is undergoing or is subsequently to undergo treatment with the angiogenesis inhibitor, in addition to the patient having undergone or undergoing or subsequently to undergo treatment with the apelin antagonist or apelin receptor antagonist.

In a further aspect, the present invention provides a pharmaceutical composition comprising an apelin antagonist (as disclosed above) and/or apelin receptor antagonist (as disclosed above), or an angiogenesis inhibitor (as disclosed above), and a Src tyrosine kinase inhibitor. This composition is preferably prepared for oral administration. Alternatively, the components can also be provided as a set or kit-of-parts, i.e. a set comprising a first pharmaceutical composition comprising an apelin antagonist and/or apelin receptor antagonist (as disclosed above), or an angiogenesis inhibitor (as disclosed above), and a second pharmaceutical composition comprising a Src tyrosine kinase inhibitor. The Src tyrosine kinase inhibitor is preferably selected from bosutinib, dasatinib, ponatinib, vandetanib, saracatinib and AZD0424, especially dasatinib. This composition or set preferably further comprises an angiogenesis inhibitor (or an apelin antagonist and/or apelin receptor antagonist) as defined herein. The composition or set can be used for any one of the therapeutic uses disclosed herein, such as treatment of breast cancer or lung cancer, e.g. in a cancer patient with resistance or at risk of developing resistance to an anti-angiogenic therapy.

In a still further aspect, the present invention relates to the Src tyrosine kinase as defined herein for use in treatment of a cancer, or in prevention or treatment of cancer metastasis, in a cancer patient. In this aspect, the patient has undergone or is undergoing or is subsequently to undergo treatment with the angiogenesis inhibitor, in addition to the patient having undergone or undergoing or subsequently to undergo treatment with the apelin antagonist or apelin receptor antagonist.

In a further aspect, the present invention provides an article of manufacture enclosing the composition or set as defined in any one of the aspects or embodiments mentioned above, together with a package insert indicating the use as defined in any one of the aspects or embodiments mentioned above.

As an exemplary embodiment, an article of manufacture (such as a packaging) encloses a pharmaceutical composition comprising an apelin antagonist and/or an apelin receptor antagonist, and further comprising an angiogenesis inhibitor, wherein the angiogenesis inhibitor is selected from the group of RTK inhibitors, together with a package insert indicating use for the treatment of a cancer patient who has resistance or is at risk of developing resistance to anti-angiogenic therapy with an angiogenesis inhibitor selected from the group of RTK inhibitors.

In a further exemplary embodiment, an article of manufacture (such as a packaging) encloses a set comprising a first pharmaceutical composition, comprising an apelin antagonist and/or an apelin receptor antagonist, and a second pharmaceutical composition, comprising an angiogenesis inhibitor, wherein the angiogenesis inhibitor is selected from the group of RTK inhibitors, together with a package insert indicating use for prevention or treatment of cancer in a patient, wherein the cancer is selected from breast cancer and lung cancer, wherein the cancer is HER-2-positive, p53-negative, ER-positive, KRas-positive or combinations thereof.

### Further definitions

The term "monoclonal antibody" as used herein refers to an antibody obtained from a population of substantially homogeneous antibodies, i.e., the individual antibodies comprising the population are identical and/or bind the same epitope, except for possible variant antibodies, e.g., containing naturally occurring mutations or arising during production of a monoclonal antibody preparation, such variants generally being present in minor amounts. In contrast to polyclonal antibody preparations, which typically include different antibodies directed against different determinants (epitopes), each monoclonal antibody of a monoclonal antibody preparation is directed against a single determinant on an antigen. Thus, the modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by a variety of techniques, including but not limited to the hybridoma method, recombinant DNA methods, phage-, yeast- or mammalian cell-display methods, and methods utilizing transgenic animals containing all or part of the human immunoglobulin loci.

The term "preventing" or "prevention" as used herein means to stop a disease state or condition from occurring in a patient or subject completely or almost completely or at least to a (preferably significant) extent, especially when the patient or subject is predisposed to such a risk of contracting a disease state or condition.

In a preference, the expression "pharmaceutical composition", especially if it comprises at least one active agent selected from anti-apelin antibodies, anti-apelin-receptor antibodies, peptide apelin antagonists or peptide apelin receptor antagonists such as apelin F13A (in an embodiment herein also called the "first pharmaceutical composition"), refers to any composition comprising the active agent and one or more excipients pharmaceutically acceptable for parenteral administration. Suitable excipients are known to the person skilled in the art, for example water (especially water for injection), saline, Ringer's solution, dextrose solution, buffers, Hank solution, vesicle forming compounds (e.g. lipids), fixed oils, ethyl oleate, 5% dextrose in saline, substances that enhance isotonicity and chemical stability, buffers and preservatives. Other suitable excipients include any compound that does not itself induce the production of antibodies in the patient that are harmful for the patient. Examples are well tolerable proteins, polysaccharides, polylactic acids, polyglycolic acid, polymeric amino acids and amino acid copolymers. This pharmaceutical composition can (as a drug) be administered via appropriate procedures known to the skilled person to a patient in need thereof (i.e. a patient having or having the risk of developing the diseases or conditions mentioned herein). The preferred route of administration of said pharmaceutical composition is parenteral administration, in particular through intravenous, intramuscular or subcutaneous administration. For parenteral administration, the pharmaceutical composition of the present invention is provided in injectable dosage unit form, eg as a solution, suspension or emulsion, formulated in conjunction with the above-defined pharmaceutically acceptable excipients. The dosage and method of administration, however, depends on the individual patient to be treated. Said pharmaceutical composition, especially comprising at least one active agent selected from anti-apelin antibodies, anti-apelin receptor antibodies, anti-apelin receptor antibodies, peptide apelin antagonists or peptide apelin receptor antagonists such as apelin F13A (in an embodiment herein also called the "first pharmaceutical composition" herein), can be administered in any suitable dosage known from other antibody or peptide dosage regimens or specifically evaluated and optimised for a given individual. For example, the active agent may be present in the pharmaceutical composition in an amount from 1 mg to 10 g, preferably 50 mg to 2 g, in particular 100 mg to 1 g. Usual dosages can also be determined on the basis of kg body weight of the patient, for example preferred dosages are in the range of 0.1 mg to 100 mg/kg body weight, especially 1 to 10 mg/kg body weight (per administration session). The administration may occur e.g. once daily, once every other day, once per week or once every two weeks. As the preferred mode of administration of said pharmaceutical composition, especially comprising at least one active agent selected from anti-apelin antibodies, anti-apelin receptor antibodies, peptide apelin antagonists or peptide apelin receptor antagonists such as apelin F13A (in an embodiment herein also called the "first pharmaceutical composition" herein), is parenteral administration, the pharmaceutical composition according to the present invention is preferably liquid or ready to be dissolved in liquid such sterile, de-ionised or distilled water or sterile isotonic phosphate-buffered saline (PBS). Preferably, 1000 µg (dry-weight) of such a composition comprises or consists of 0.1-990 µg, preferably 1-900µg, more preferably 10- 200µg apelin (receptor) antagonist, and optionally 1-500 µg, preferably 1-100 µg, more preferably 5-15 µg (buffer) salts (preferably to yield an isotonic buffer in the final volume), and optionally 0.1-999.9 µg, preferably 100-999.9 µg, more preferably 200-999 µg other excipients. Preferably, 100 mg of such a dry composition is dissolved in sterile, de-ionised/distilled water or sterile isotonic phosphate-buffered saline (PBS) to yield a final volume of 0.1-100 ml, preferably 0.5-20 ml, more preferably 1-10 ml.

In a further preference, the expression "pharmaceutical composition", especially if it comprises at least one active agent selected from RTK inhibitors such as sunitinib, sorafenib, pazopanib, axitinib, cabozantinib, regorafenib, vandetanib, tivozanib, cediranib, nintedanib and vatalanib (in an embodiment herein also called the "second pharmaceutical composition"), refers to any composition comprising the active agent and one or more excipients pharmaceutically acceptable for oral administration. Suitable excipients are known to the person skilled in the art, e.g. inert diluents, such as calcium carbonate, sodium carbonate, lactose, calcium phosphate or sodium phosphate; granulating and disintegrating agents, e.g. corn starch, or alginic acid; binding agents, e.g. starch, gelatin or acacia, and lubricating agents, e.g. magnesium stearate, stearic acid or talc. The pharmaceutical composition may be presented e.g. in tablet or capsule form. The preferred route of administration of said pharmaceutical composition is oral administration. For example, the active agent may be present in the pharmaceutical composition in an amount from 0.1 mg to 10 g, preferably 1 mg to 250 mg, in particular 10 mg to 100 mg. Usual dosages can also be determined on the basis of kg body weight of the patient, for example preferred dosages are in the range of 0.01 mg to 10 mg/kg body weight, especially 0.1 to 1 mg/body weight (per administration session). The administration may occur e.g. once daily, once every other day, once per week or once every two weeks, especially once daily.

It is evident to the skilled person that active agents described herein can also be administered in salt-form (i.e. as a pharmaceutically acceptable salt of the active agent, such as for instance sunitinib malate). Accordingly, any mention of an active agent herein shall also include any pharmaceutically acceptable salt forms thereof.

Herein, the term "specific for" - as in "molecule A specific for molecule B" - means that molecule A has a binding preference for molecule B compared to other molecules in a patient's body. Typically, this entails that molecule A (such as an antibody) has a dissociation constant (also called "affinity") in regard to molecule B (such as the antigen, specifically the binding epitope thereof) that is lower than (i.e. "stronger than") 1000 nM, preferably lower than 100 nM, more preferably lower than 50 nM, even more preferably lower than 10 nM, especially lower than 5 nM.

Preferably, the patient is a mammal, especially a human (male or female). Typically, the patient (who preferably has a cancer as defined herein, and especially has been diagnosed with a cancer as defined herein) is in need of the inventive treatment. In the context of the inventive treatment for prevention of a cancer, the patient may be a patient that does not have the cancer but is at risk of developing the cancer.

Herein, the terms "mammary cancer" and "breast cancer" are used interchangeably. A patient having a malignant "mammary tumour" is equivalent to the patient having breast cancer.

Herein, the expressions "compound X is administered before compound Y is administered" and "compound X is administered after compound Y has been administered", with compound X being e.g. the apelin antagonist or apelin receptor antagonist and compound Y being e.g. the RTK inhibitor, or similar expressions, mean that a compound X and a compound Y are administered separately, in a temporal sequence. By way of example, a course of treatment with compound X ends (or starts, respectively) within e.g. one day, one week, two weeks, three weeks, one month, two months, three months, six months, one year, or two years before the course of treatment with compound Y starts (or after the course of treatment with compound Y has ended, respectively). Yet, within the scope of said two expressions, the courses of treatment may also overlap.

Herein, the term "over-expressing" or similar in regard to a gene (product) A typically can mean that the expression level of A (as e.g. measured by Western blot or immunohistochemistry) e.g. in a tumour sample is higher than the expression level of an appropriate control (such as healthy tissue of the same type), by a factor of at least 1.2 (i.e. an increase of at least 20%), preferably at least 1.4, more preferably at least 1.6, even more preferably at least 1.8, especially at least 2.0; e.g. in connection with oncogene status such as status of HER-2, ER and Kras.

Herein, the term "under-expressing" or similar in regard to a gene (product) A typically can mean that the expression level of A (as e.g. measured by Western blot or immunohistochemistry) e.g. in a tumour sample is lower than the expression level of an appropriate control (such as healthy tissue of the same type), by a factor of at most 0.8 (i.e. a decrease of at least 20%), preferably at most 0.7, more preferably at most 0.6, even more preferably at most 0.5, especially at most 0.25 or even at most 0.1; e.g. in connection with tumour suppressor status such as p53 status.

The present invention further relates to the following embodiments:
Embodiment 1. A pharmaceutical composition comprising an apelin antagonist and/or an apelin receptor antagonist, and further comprising an angiogenesis inhibitor, wherein the angiogenesis inhibitor is selected from the group of receptor tyrosine kinase (RTK) inhibitors.
Embodiment 2. A set comprising a first pharmaceutical composition, comprising an apelin antagonist and/or an apelin receptor antagonist, and a second pharmaceutical composition, comprising an angiogenesis inhibitor, wherein the angiogenesis inhibitor is selected from the group of RTK inhibitors.
Embodiment 3. The composition or set according to embodiment 1 or 2, wherein the angiogenesis inhibitor is selected from the group of sunitinib, sorafenib, pazopanib, axitinib, cabozantinib, regorafenib, vandetanib, tivozanib, cediranib, nintedanib and vatalanib.
Embodiment 4. The composition or set according to embodiment 3, wherein the angiogenesis inhibitor is sunitinib.
Embodiment 5. The composition or set according to any one of embodiments 1 to 4, wherein the apelin antagonist is selected from the group of anti-apelin antibodies, and/or
   wherein the apelin receptor antagonist is selected from apelin analogues, preferably a peptide with the sequence QRPRLSHKGPMPA (SEQ ID NO: 14) or N-α-acetyl-nona-D-arginine amide (SEQ ID NO: 22) or cyclo(1-6)CRPRLC-KH-cyclo(9-14)CRPRLC (SEQ ID NO: 23), and anti-apelin-receptor antibodies.
Embodiment 6. An apelin antagonist and/or apelin receptor antagonist for use in the prevention or treatment of cancer in a patient, wherein the apelin antagonist and/or apelin receptor antagonist is administered to the patient, and
   wherein an angiogenesis inhibitor, preferably selected from the group of RTK inhibitors, is administered to the patient, in a composition together with the apelin antagonist and/or apelin receptor antagonist, in combination with the apelin antagonist and/or apelin receptor antagonist, before the apelin antagonist and/or apelin receptor antagonist is administered, or after the apelin antagonist and/or apelin receptor antagonist has been administered.
Embodiment 7. An angiogenesis inhibitor for use in the prevention or treatment of cancer in a patient, wherein the angiogenesis inhibitor is administered to the patient, and
   wherein an apelin antagonist and/or apelin receptor antagonist, is administered to the patient, in a composition together with the angiogenesis inhibitor, in combination with the angiogenesis inhibitor, before the angiogenesis inhibitor is administered, or after the angiogenesis inhibitor has been administered,
   preferably wherein the angiogenesis inhibitor is selected from the group of RTK inhibitors.
Embodiment 8. An apelin antagonist and/or apelin receptor antagonist for use in the prevention or treatment of metastasis in a cancer patient, wherein the patient has undergone or is undergoing cancer treatment with an angiogenesis inhibitor, preferably selected from the group of RTK inhibitors.
Embodiment 9. An angiogenesis inhibitor for use in the prevention or treatment of metastasis in a cancer patient, wherein the patient has undergone or is undergoing cancer treatment with an apelin antagonist and/or apelin receptor antagonist,
   preferably wherein the angiogenesis inhibitor is selected from the group of RTK inhibitors.
Embodiment 10. An apelin antagonist and/or apelin receptor antagonist for use in the treatment of a cancer patient who has resistance or is at risk of developing resistance to anti-angiogenic therapy, preferably anti-angiogenic therapy with an angiogenesis inhibitor selected from the group of RTK inhibitors.
Embodiment 11. An angiogenesis inhibitor for use in the treatment of a cancer patient who has resistance or is at risk of developing resistance to anti-angiogenic therapy, wherein the angiogenesis inhibitor is administered to the patient, and
   wherein an apelin antagonist and/or apelin receptor antagonist, is administered to the patient, in a composition together with the angiogenesis inhibitor, in combination with the angiogenesis inhibitor, before the angiogenesis inhibitor is administered, or after the angiogenesis inhibitor has been administered,
   preferably wherein the angiogenesis inhibitor is selected from the group of RTK inhibitors.
Embodiment 12. The apelin antagonist and/or apelin receptor antagonist, or the angiogenesis inhibitor, for use according to any one of embodiments 6 to 11, wherein the angiogenesis inhibitor is selected from the group of sunitinib, sorafenib, pazopanib, axitinib, cabozantinib, regorafenib, vandetanib, tivozanib, cediranib, nintedanib and vatalanib.
Embodiment 13. The apelin antagonist and/or apelin receptor antagonist, or the angiogenesis inhibitor, for use according to embodiment 12, wherein the angiogenesis inhibitor is sunitinib.
Embodiment 14. The apelin antagonist and/or apelin receptor antagonist, or the angiogenesis inhibitor, for use according to any one of embodiments 6 to 13, wherein the apelin antagonist is selected from the group of anti-apelin antibodies and/or,
   wherein the apelin receptor antagonist is selected from apelin analogues, preferably a peptide with the sequence QRPRLSHKGPMPA (SEQ ID NO: 14) or N-α-acetyl-nona-D-arginine amide (SEQ ID NO: 22) or cyclo(1-6)CRPRLC-KH-cyclo(9-14)CRPRLC (SEQ ID NO: 23), and anti-apelin-receptor antibodies.
Embodiment 15. The apelin antagonist and/or apelin receptor antagonist, or the angiogenesis inhibitor, for use according to any one of embodiments 6 to 14, wherein the cancer is of epithelial origin and/or selected from breast cancer, lung cancer ovarian cancer, cervical cancer, hepatocellular carcinoma, renal cell carcinoma, thyroid cancer, neuroendocrine cancer, gastro-oesophageal cancer, colorectal cancer, glioma, prostate cancer and pancreatic cancer, in particular selected from breast cancer and lung cancer.
Embodiment 16. The apelin antagonist and/or apelin receptor antagonist, or the angiogenesis inhibitor, for use according to any one of embodiments 6 to 15, wherein the cancer is HER-2-positive, estrogen-receptor-positive, p53-negative, KRas-positive or combinations thereof.
Embodiment 17. The apelin antagonist and/or apelin receptor antagonist, or the angiogenesis inhibitor, for use according to any one of embodiments 6 to 16, wherein the cancer over-expresses epithelial-mesenchymal transition (EMT) markers, preferably selected from the group of Snail, fibronectin, and TGF-beta, and/or
   wherein the cancer over-expresses invasiveness-related genes, preferably selected from the group of Met, IL1b, COL1A1 and COL1A2 and/or
   wherein the cancer over-expresses apelin and/or apelin receptor.
Embodiment 18. The apelin antagonist and/or apelin receptor antagonist for use according to any one of embodiments 6 to 17, wherein a further chemotherapeutic agent, and/or radiotherapy for the latter three of the following cases, is administered to the patient, in a composition together with the apelin antagonist and/or apelin receptor antagonist, in combination with the apelin antagonist and/or apelin receptor antagonist, before the apelin antagonist and/or apelin receptor antagonist is administered, or after the apelin antagonist and/or apelin receptor antagonist has been administered,
   wherein the further chemotherapeutic agent is selected from the group of alkylating agents, platin-based chemotherapeutic agents, antimetabolites, anti-microtubule agents, topoisomerase inhibitors and cytotoxic antibiotics,
   preferably wherein the further chemotherapeutic agent is selected from the group of 5-fluorouracil, cisplatin, carboplatin, cyclophosphamide, doxorubicin, epirubicin, gemcitabine, taxanes such as paclitaxel or docetaxel, methotrexate, pemetrexed, vinorelbine and etoposide, in particular etoposide.
Embodiment 19. The angiogenesis inhibitor for use according to any one of embodiments 6 to 17, wherein a further chemotherapeutic agent, and/or radiotherapy for the latter three of the following cases, is administered to the patient, in a composition together with the angiogenesis inhibitor, in combination with the angiogenesis inhibitor, before the angiogenesis inhibitor is administered, or after the angiogenesis inhibitor has been administered,
   wherein the further chemotherapeutic agent is selected from the group of alkylating agents, platin-based chemotherapeutic agents, antimetabolites, anti-microtubule agents, topoisomerase inhibitors and cytotoxic antibiotics,
   preferably wherein the further chemotherapeutic agent is selected from the group of 5-fluorouracil, cisplatin, carboplatin, cyclophosphamide, doxorubicin, epirubicin, gemcitabine, taxanes such as paclitaxel or docetaxel, methotrexate, pemetrexed, vinorelbine and etoposide, in particular etoposide, and/or preferably wherein the angiogenesis inhibitor is selected from the group of RTK inhibitors such as sunitinib.
Embodiment 20. The apelin antagonist and/or apelin receptor antagonist for use according to any one of embodiments 6 to 18, wherein a Src tyrosine kinase inhibitor is also administered to the patient, in a composition together with the apelin antagonist and/or apelin receptor antagonist, in combination with the apelin antagonist and/or apelin receptor antagonist, before the apelin antagonist and/or apelin receptor antagonist is administered or after the apelin antagonist and/or apelin receptor antagonist has been administered,
   preferably wherein the Src tyrosine kinase inhibitor is selected from bosutinib, dasatinib, ponatinib, vandetanib, saracatinib and AZD0424, especially dasatinib.
Embodiment 21. The angiogenesis inhibitor for use according to any one of embodiments 6 to 19, wherein a Src tyrosine kinase inhibitor is also administered to the patient, in a composition together with the angiogenesis inhibitor, in combination with the angiogenesis inhibitor, before the angiogenesis inhibitor is administered or after the angiogenesis inhibitor has been administered,
   preferably wherein the Src tyrosine kinase inhibitor is selected from bosutinib, dasatinib, ponatinib, vandetanib, saracatinib and AZD0424, especially dasatinib and/or
   preferably wherein the angiogenesis inhibitor is selected from the group of RTK inhibitors such as sunitinib.
Embodiment 22. The apelin antagonist and/or apelin receptor antagonist, or the angiogenesis inhibitor, for use according to any one of embodiments 6 to 21, wherein the patient has an apelin level, preferably a plasma or serum apelin level, higher than a control, and/or the cancer has an apelin or apelin receptor level higher than a control;
   preferably wherein the apelin level is measured after the patient has undergone cancer treatment with an angiogenesis inhibitor, preferably selected from the group of RTK inhibitors, especially sunitinib; and/or
   preferably wherein the cancer is selected from breast cancer, lung cancer, ovarian cancer, cervical cancer, hepatocellular carcinoma, renal cell carcinoma, thyroid cancer, neuroendocrine cancer, gastro-oesophageal cancer, colorectal cancer, glioma, prostate cancer and pancreatic cancer, in particular selected from breast cancer and lung cancer.
Embodiment 23. The apelin antagonist and/or apelin receptor antagonist, or the angiogenesis inhibitor, for use according to any one of embodiments 6 to 22, for tumour blood vessel normalisation.
Embodiment 24. A pharmaceutical composition comprising the apelin antagonist and/or apelin receptor antagonist as set forth in any one of embodiments 1 to 23 and a further chemotherapeutic agent,
   wherein the further chemotherapeutic agent is selected from the group of alkylating agents, platin-based chemotherapeutic agents, antimetabolites, anti-microtubule agents, topoisomerase inhibitors and cytotoxic antibiotics, preferably selected from the group of 5-fluorouracil, cisplatin, carboplatin, cyclophosphamide, doxorubicin, epirubicin, gemcitabine, taxanes such as paclitaxel or docetaxel, methotrexate, pemetrexed, vinorelbine and etoposide, in particular etoposide,
   preferably wherein the composition is for parenteral, especially intravenous, administration.
Embodiment 25. A set comprising a first pharmaceutical composition comprising the apelin antagonist and/or apelin receptor antagonist as set forth in any one of embodiments 1 to 23 and a second pharmaceutical composition comprising a further chemotherapeutic agent,
   wherein the further chemotherapeutic agent is selected from the group of alkylating agents, platin-based chemotherapeutic agents, antimetabolites, anti-microtubule agents, topoisomerase inhibitors and cytotoxic antibiotics, preferably selected from the group of 5-fluorouracil, cisplatin, carboplatin, cyclophosphamide, doxorubicin, epirubicin, gemcitabine, taxanes such as paclitaxel or docetaxel, methotrexate, pemetrexed, vinorelbine and etoposide, in particular etoposide.
Embodiment 26. A pharmaceutical composition comprising the apelin antagonist and/or apelin receptor antagonist as set forth in any one of embodiments 1 to 23, and a Src tyrosine kinase inhibitor, preferably selected from bosutinib, dasatinib, ponatinib, vandetanib, saracatinib and AZD0424, especially dasatinib, preferably wherein the composition is for oral administration.
Embodiment 27. A set comprising a first pharmaceutical composition comprising the apelin antagonist and/or apelin receptor antagonist as set forth in any one of embodiments 1 to 23 and a second pharmaceutical composition comprising a Src tyrosine kinase inhibitor,
   preferably selected from bosutinib, dasatinib, ponatinib, vandetanib, saracatinib and AZD0424, especially dasatinib.
Embodiment 28. The pharmaceutical composition of embodiment 24 or 26, further comprising the angiogenesis inhibitor as set forth in any one of embodiments 1 to 23,
   preferably wherein the angiogenesis inhibitor is an RTK inhibitor such as sunitinib.
Embodiment 29. The set of embodiment 25 or 27, further comprising the angiogenesis inhibitor as set forth in any one of embodiments 1 to 23, wherein the angiogenesis inhibitor is present in the first pharmaceutical composition, in the second pharmaceutical composition or in a third pharmaceutical composition,
   preferably wherein the angiogenesis inhibitor is an RTK inhibitor such as sunitinib.
Embodiment 30. A pharmaceutical composition comprising the angiogenesis inhibitor as set forth in any one of embodiments 1 to 23, and a Src tyrosine kinase inhibitor, preferably selected from bosutinib, dasatinib, ponatinib, vandetanib, saracatinib and AZD0424, especially dasatinib;
   preferably wherein the composition is for oral administration, and/or
   preferably for use in any one of the uses set forth in embodiments 6 to 23 such as prevention or treatment of metastasis, wherein the apelin antagonist or apelin receptor antagonist as set forth in any one of embodiments 1 to 23 is also administered or has been administered or is subsequently to be administered to the patient, especially wherein the apelin antagonist is an anti-apelin antibody or the apelin receptor antagonist is an anti-apelin-receptor antibody.
Embodiment 31. A set comprising the angiogenesis inhibitor as set forth in any one of embodiments 1 to 23 and a second pharmaceutical composition comprising a Src tyrosine kinase inhibitor preferably selected from bosutinib, dasatinib, ponatinib, vandetanib, saracatinib and AZD0424, especially dasatinib; preferably for use in any one of the uses set forth in embodiments 6 to 23 such as prevention or treatment of metastasis, wherein the apelin antagonist or apelin receptor antagonist as set forth in any one of embodiments 1 to 23 is also administered or has been administered or is subsequently to be administered to the patient, especially wherein the apelin antagonist is an anti-apelin antibody or the apelin receptor antagonist is an anti-apelin-receptor antibody.
Embodiment 32. The pharmaceutical composition of embodiment 30, further comprising the apelin antagonist and/or apelin receptor antagonist as set forth in any one of embodiments 1 to 23.
Embodiment 33. The set of embodiment 31, further comprising the apelin antagonist and/or apelin receptor antagonist as set forth in any one of embodiments 1 to 23, wherein the apelin antagonist and/or apelin receptor antagonist is present in the first pharmaceutical composition, in the second pharmaceutical composition or in a third pharmaceutical composition,
   preferably wherein the apelin antagonist is an anti-apelin antibody or the apelin receptor antagonist is an anti-apelin-receptor antibody.
Embodiment 34. The composition or set of any one of embodiments 30 to 33, wherein the angiogenesis inhibitor is an RTK inhibitor, preferably selected from the group of sunitinib, sorafenib, pazopanib, axitinib, cabozantinib, regorafenib, vandetanib, tivozanib, cediranib, nintedanib and vatalanib, especially sunitinib.
Embodiment 35. The composition or set of embodiment 34, wherein the angiogenesis inhibitor sunitinib and the Src tyrosine kinase inhibitor is dasatinib.
Embodiment 36. The composition or set of any one of embodiments 24 to 35 for use in any one of the uses set forth in embodiments 6 to 23.
Embodiment 37. The composition or set for use according to embodiment 36, for use in the prevention or treatment of a cancer,
   preferably wherein the cancer is selected from breast cancer and lung cancer.
Embodiment 38. The composition or set for use according to embodiment 36, for use in the prevention or treatment of metastasis in a cancer patient, wherein the patient has undergone or is undergoing cancer treatment with an angiogenesis inhibitor, preferably selected from the group of RTK inhibitors such as sunitinib,
   preferably wherein the cancer is selected from breast cancer and lung cancer.
Embodiment 39. The composition or set for use according to embodiment 36, for use in the treatment of a cancer patient who has resistance or is at risk of developing resistance to anti-angiogenic therapy, preferably anti-angiogenic therapy with an angiogenesis inhibitor selected from the group of RTK inhibitors such as sunitinib,
   preferably wherein the cancer is selected from breast cancer and lung cancer.
Embodiment 40. A chemotherapeutic agent selected from the group of alkylating agents, platin-based chemotherapeutic agents, antimetabolites, anti-microtubule agents, topoisomerase inhibitors and cytotoxic antibiotics, especially topoisomerase inhibitors,
   for use in treatment of a cancer, or in prevention or treatment of cancer metastasis, in a cancer patient,
   wherein the patient has undergone or is undergoing or is to subsequently undergo treatment with an angiogenesis inhibitor, the angiogenesis inhibitor as set forth in any one of embodiments 1 to 23, and wherein the patient has undergone or is undergoing or is to subsequently undergo treatment with an apelin antagonist or apelin receptor antagonist, either antagonist as set forth in any one of embodiments 1 to 23,
   preferably wherein the patient has undergone said treatment with the angiogenesis inhibitor and said treatment with either antagonist, or wherein the patient is undergoing said treatment with the angiogenesis inhibitor and said treatment with either antagonist.
Embodiment 41. The chemotherapeutic agent for use according to embodiment 40, wherein the agent is selected from the group of 5-fluorouracil, cisplatin, carboplatin, cyclophosphamide, doxorubicin, epirubicin, gemcitabine, taxanes such as paclitaxel or docetaxel, methotrexate, pemetrexed, vinorelbine and etoposide, in particular etoposide.
Embodiment 42. The chemotherapeutic agent for use according to embodiment 40 or 41, wherein the angiogenesis inhibitor is selected from the group of RTK inhibitors, preferably from the group of sunitinib, sorafenib, pazopanib, axitinib, cabozantinib, regorafenib, vandetanib, tivozanib, cediranib, nintedanib and vatalanib, especially sunitinib.
Embodiment 43. The chemotherapeutic agent for use according to any one of embodiments 40 to 42, wherein the apelin antagonist is selected from the group of anti-apelin antibodies, or wherein the apelin receptor antagonist is selected from apelin analogues and anti-apelin-receptor antibodies.
Embodiment 44. The chemotherapeutic agent for use according to any one of embodiments 40 to 43, wherein the cancer is selected from breast cancer, lung cancer, ovarian cancer, cervical cancer, hepatocellular carcinoma, renal cell carcinoma, thyroid cancer, neuroendocrine cancer, gastro-oesophageal cancer, colorectal cancer, glioma, prostate cancer and pancreatic cancer, in particular selected from breast cancer and lung cancer.
Embodiment 45. A Src tyrosine kinase inhibitor for use in treatment of a cancer, or in prevention or treatment of cancer metastasis, in a cancer patient,
   wherein the patient has undergone or is undergoing or is to subsequently undergo treatment with an angiogenesis inhibitor, the angiogenesis inhibitor as set forth in any one of embodiments 1 to 23, and wherein the patient has undergone or is undergoing or is to subsequently undergo treatment with an apelin antagonist or apelin receptor antagonist, either antagonist as set forth in any one of embodiments 1 to 23,
   preferably wherein the patient has undergone said treatment with the angiogenesis inhibitor and said treatment with either antagonist, or wherein the patient is undergoing said treatment with the angiogenesis inhibitor and said treatment with either antagonist.
Embodiment 46. The Src tyrosine kinase inhibitor for use according to embodiment 45, wherein the Src tyrosine kinase inhibitor is selected from bosutinib, dasatinib, ponatinib, vandetanib, saracatinib and AZD0424, especially dasatinib.
Embodiment 47. The Src tyrosine kinase inhibitor for use according to embodiment 45 or 46, wherein the angiogenesis inhibitor is selected from the group of RTK inhibitors, preferably from the group of sunitinib, sorafenib, pazopanib, axitinib, cabozantinib, regorafenib, vandetanib, tivozanib, cediranib, nintedanib and vatalanib, especially sunitinib.
Embodiment 48. The Src tyrosine kinase inhibitor for use according to any one of embodiments 45 to 47, wherein the apelin antagonist is selected from the group of anti-apelin antibodies, or wherein the apelin receptor antagonist is selected from apelin analogues and anti-apelin-receptor antibodies.
Embodiment 49. The Src tyrosine kinase inhibitor according to any one of embodiments 45 to 48, wherein the cancer is selected from breast cancer, lung cancer, ovarian cancer, cervical cancer, hepatocellular carcinoma, renal cell carcinoma, thyroid cancer, neuroendocrine cancer, gastro-oesophageal cancer, colorectal cancer, glioma, prostate cancer and pancreatic cancer, in particular selected from breast cancer and lung cancer.

The present invention is further illustrated by the following figures and examples, without being restricted thereto.
**Fig. 1****:** Human apelin variants. The human APLN gene codes for an apelin preproprotein ("apelin-77") containing a signal peptide in its amino-terminal sequence thus directing apelin in the secretory pathway. Eventually the proprotein ("apelin-55") gives rise to several bioactive carboxy-terminal fragments including apelin-36, apelin-17 and apelin-13. The amino-terminal glutamine of apelin-13 can be post-translationally modified thus creating the pyroglutamate apelin-13 (pyr-apelin-13) which is more protected from exopeptidase degradation.
**Fig. 2****:** Apelin is a prognostic factor in breast and lung cancer. a) Kaplan Meier plots for high and low Apelin-expressing groups in human breast cancer, data from the KM-plotter database. b) Kaplan Meier plots for high and low Apelin-expressing groups in human breast cancer, from the Prognoscan database. 95% confidence intervals for each group are indicated by dotted lines. c) Kaplan Meier plots for high and low Apelin-expressing groups in human NSCLC (non-squamous cell lung cancer), from the Prognoscan database. 95% confidence intervals for each group are indicated by dotted lines.
**Fig. 3****:** Combination of apelin ablation and anti-angiogenic treatment with an RTK inhibitor further increased survival and reduced tumour burden in breast cancer. a) Scheme of the experimental design followed. Anti-angiogenic treatment with sunitinib malate (60mg/kg per dose by gavage, three times a week) was started only after cancer onset, established by mammary tumour palpation and followed until the mice were sacrificed when the tumour size was 1 cm³, following ethical guidelines. b) Kaplan Meier plot for survival in NeuT; Apln^{+/+} and NeuT; Apln^{-/-} mice with mammary cancer, control or treated with sunitinib malate after tumour onset (*P<0.05; Log rank test; n=7-11). Mice were sacrificed when the tumour size was 1 cm³, following ethical guidelines. c) Percentage of proliferative area in age-matched NeuT;Apln^{+/+} and NeuT;Apln^{-/-} mammary glands (*P<0.05; T test) from control mice or mice treated with sunitinib malate (60mg/kg per dose) three times a week (n=3-7). Mammary glands were H&E stained and analysed in a blinded and automated fashion.
**Fig. 4****:** Combination of apelin ablation and anti-angiogenic treatment with an RTK inhibitor further increased survival and reduced tumour burden in lung cancer. a) Scheme of the experimental design followed. Anti-angiogenic treatment with sunitinib malate (60mg/kg per dose by gavage, three times a week) was started at cancer onset, three weeks after Ad-Cre intratracheal delivery. b) Kaplan Meier plot for survival in p53^{f/f};KRas;Apln^{+/+} and p53^{f/f};KRas;Apln^{-/-} mice with lung cancer, control or treated with Sunitinib Malate after tumour onset (* P<0.05; Log rank test; n=10-12). c) Percentage of proliferative area in age-matched p53^{f/f};KRas;Apln^{+/+} and p53^{f/f};KRas;Apln^{-/-} mice lungs (*P<0.05; T test) from control mice or mice treated with sunitinib malate (60 mg/kg per dose) three times a week (n=7-9). Lungs were H&E stained and analysed in a blinded and automated fashion.

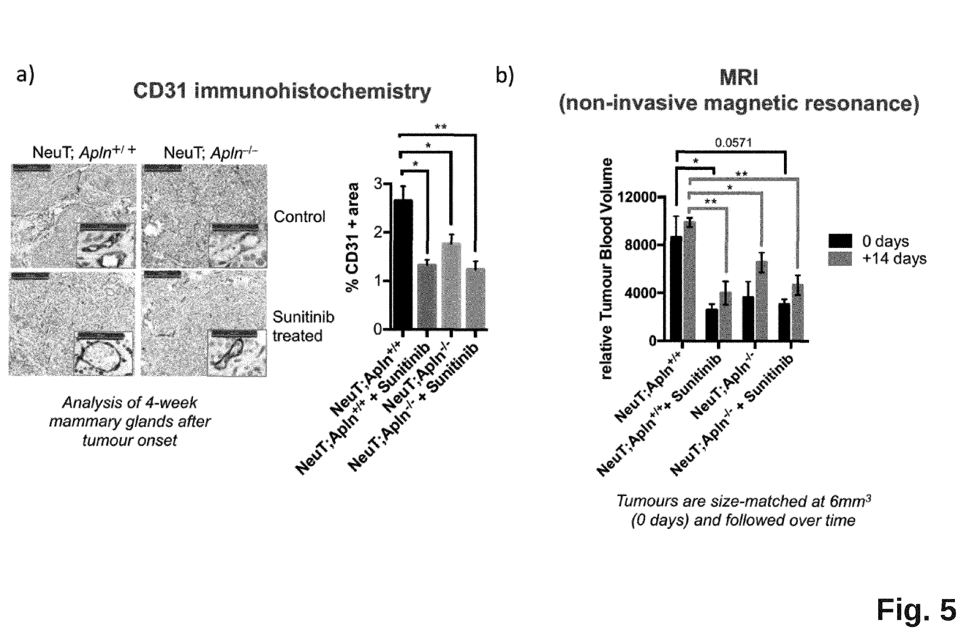

**Fig. 5****:** Combination of apelin ablation and anti-angiogenic treatment with an RTK inhibitor did not further reduce angiogenesis. a) CD31-positive area (percentage) in NeuT;Apln^{+/+} and NeuT;Apln^{-/-} age-matched mammary tumours control or treated with sunitinib malate 4 weeks after tumour onset (*P<0.05; T-test; n=6). b) Relative tumour blood volume quantification from non-invasive MRI data following over-time NeuT;Apln^{+/+} and NeuT;Apln^{-/-}mammary tumours, control or treated with sunitinib malate. Tumours were size-matched at 30-50mm³ (called "0 days") and followed over time.

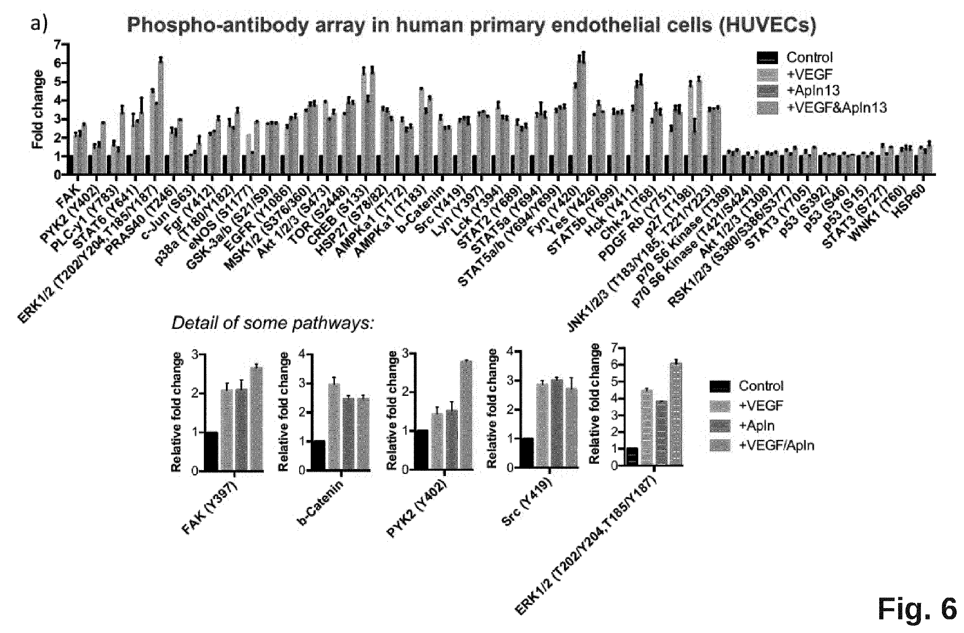

**Fig. 6****:** Apelin regulated adhesion pathways similarly to VEGF. a) Phospho-antibody array in primary human umbilical venous endothelial cells (HUVECs) stimulated with VEGF, apelin-13 or both.
**Fig. 7****:** Combination of apelin ablation and anti-angiogenic treatment with an RTK inhibitor reduced vessel leakage and interstitial pressure, thereby leading to vessel normalisation. Quantification from non-invasive MRI data following over-time NeuT; Apl^{n+/+} and NeuT;Apln^{-/-} mammary tumours, control or treated with sunitinib malate. Tumours were size-matched and followed over-time to study a) K₂ leakage pre/post contrast and b) T₂.
**Fig. 8****:** Combination of apelin ablation and anti-angiogenic treatment with an RTK inhibitor reduced vessel leakage also in lung cancer, thereby leading to vessel normalisation. a) Quantification of the number of CD31+ vessels with extravasated Dextran in age-matched p53^{f/f};KRas;Apln^{+/+} and pS3^{f/f}; KRas; Apln^{-/-}mice with lung cancer, control or treated with sunitinib malate after tumour onset.

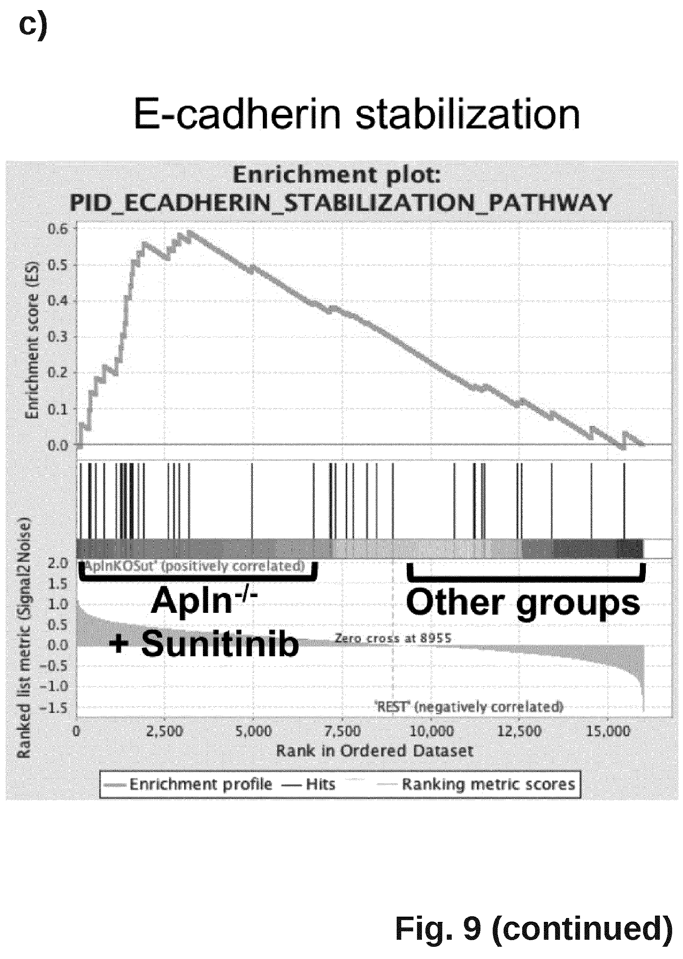

**Fig. 9****:** Combination of apelin ablation and anti-angiogenic treatment with an RTK inhibitor reduced tumour proliferation, malignancy, EMT and invasiveness. Size-matched large mammary tumours of 1 cm³ analysed for: a) Ki67-positive nuclei quantification and representative images. The tumours were analysed in a blinded and automated fashion (*P<0.05; T test; n=5-6). b) qRT-PCR of EMT markers (Snail, FN1, Tgf-beta) and invasiveness-related genes (Met, IL1b, COL1A1, COL1A2). Significance is shown compared to NeuT;Apln^{+/+} controls (*P<0.05; T test). c, d, e) Gene set enrichment analysis (GSEA) from RNA-seq samples comparing Sunitinib-treated NeuT;Apln^{-/-} tumours to the other tumours (NeuT;Apln^{+/+} control, NeuT;Apln^{-/-} control and Sunitinib-treated NeuT; Apln^{+/+}) .
**Fig. 10****:** Targeting apelin reduced metastasis induced by anti-angiogenic treatment with an RTK inhibitor. a) Primary tumour growth of E0771 mammary cancer cells interfered with control shRenilla or with shApelin and injected orthotopically in the mammary gland of B6 mice, either Apln^{+/+} (n=3) or Apln^{-/-} (n=3) . b) Quantification of lung metastatic foci of E0771 mammary cancer cells. Mice were sacrificed when the primary tumour was between 1-1.5 cm³ following ethical guidelines.

### Examples

### Example 1 - Materials & methods

The following materials & methods were used in subsequent examples 2-6.

Apelin knockout mice: These mice were generated previously and carry a deletion of the apelin gene in the germline (Kuba et al., 2007).

Breast cancer models: MMTV-NeuT transgenic mice were used, which carry the activated c-neu oncogene, a major human breast cancer oncogene, driven by a mouse mammary tumour virus (MMTV) promoter (Muller et al., 1988). These transgenic mice develop mammary adenocarcinomas that involve the entire epithelium in each gland and are widely used as a model to study breast cancer. Tumour onset of mammary tumours was determined by weekly palpation of the mammary glands. In addition, the E0771 metastatic mammary cancer model was also used (Ewens, et al., 2005).

Lung cancer models: Cre-inducible model of K-RasG12D-driven non-small cell lung cancer (NSCLC) combined with p53 floxed (Jackson, 2001), the most prevalent form of lung cancer. The expression of K-RasG12D is controlled by a Lox-Stop-Lox cassette, which can be removed by Cre recombinase. Intratracheal administration of adeno- or lenti-viruses expressing Cre was used to specifically induce K-RasG12D expression and p53 deletion in the pneumocytes of the lung (DuPage et al., 2009a).

Anti-angiogenic treatment with the multimodal RTK inhibitor sunitinib malate (60mg/kg body weight per dose by oral gavage, three times a week) was started only after cancer onset and followed until the mice were sacrificed.

Animals were genotyped by PCR analysis of genomic DNA. Mice were maintained in temperature-controlled conditions. All animal experiments were carried out in agreement with the ethical animal license protocol in accordance with the current laws of Austria.

Histology, whole-mount and immunohistochemistry analysis: Tissue samples were fixed in 4% paraformaldehyde (PFA) overnight at 4°C and embedded in paraffin after dehydration in ascending concentrations of ethanol. For histological analysis, 2-4µm-thick paraffin sections were prepared and stained with haematoxylin and eosin and scanned with a Mirax slide scanner. The proliferative tumour area was automatically scored by an algorithm programmed and executed using the Definiens software suite program and visually controlled by a pathologist in a blinded fashion. Determination of proliferative tissue in histologic sections was performed in accordance with the mouse mammary pathology consensus. For histological analysis of lung tumours, sections from at least 3 different central planes of the lungs were cut and analysed. For immunohistochemistry, the automated Ventana system was used. Paraffin sections were stained with antibodies against Ki67 (Novacastra) and CD31 (Dianova).

Vascular permeability assay: Lysinated labelled dextran (70kDa; Invitrogen, D1818, 1.25mg in 100uL ddH2O) was intravenously injected into the tail vein of each tumour-bearing mouse. At 15 minutes post-injection, mice were sacrificed and tumours were harvested, followed by overnight fixation in 4%PFA. Whole-mount immunostaining of tumour samples was performed with anti-CD31 (Dianova) followed by goat Alexa 633 secondary antibody. Positive signals were examined by confocal microscopy.

Non-invasive MRI: MRI was performed on a 15.2 T Bruker system (Bruker BioSpec, Ettlingen Germany) with a 35 mm quadrature birdcage coil. Mice were continuously monitored for tumour occurrence by palpation and MRI. When tumours reached 30-50 mm³ this was set as imaging day 0, and then each tumour was imaged after 2, 4 and 6 weeks. Before imaging, the tail line was inserted for delivery of contrast agent (30 gauge needle with silicon tubing). All animals were anaesthetised with isoflurane (4% induction, maintenance with 1.5%). During imaging, respiration was monitored and isoflurane levels were adjusted if breathing was <20 or >60 breaths per minute. Mice were kept warm with water heated to 37°C circulated using a water pump. For measurement of tumour volume and transverse relaxation time (T2)-quantification, a multi-slice multi-echo (MSME) spin echo sequence was used (repetition time (TR)/echo time (TE) = 3000/5.8-81.18 ms, 14 echoes, 117 µm2 in-plane resolution, 0.5 mm slice thickness, number of experiments [NEX] = 1). The tumour volume was calculated by multiplying the slice thickness with the tumour area by an investigator blinded to the treatment group. ImageJ was used to calculate T2-values. Dynamic susceptibility contrast (DSC) perfusion MRI was collected using fast imaging with steady-state precession (FISP) with 500.6 ms temporal resolution (1 slice; TR/TE = 500/1.7 ms; flip-angle = 5 degree; 4692 µm² in-plane resolution; 1-mm slice thickness; NEX = 2; 360 repetitions) following tail-vein injection of 0.05 mL of 0.25 mol/L gadolinium-based contrast agent (Magnevist, Berlex). Prior to and following DSC-MRI, a T1-weighted spin-echo dataset was acquired (0.5 mm thick slices, TR/TE 500/5.8 ms, 117 µm² resolution, 2 NEX).

Perfusion data were processed offline using ImageJ (National Institutes of Health; rsbweb.nih.gov/ij/), and the DSCoMAN plugin (Duke University, https://dblab.duhs.duke.edu/wysiwyg/downloads/DSCoMAN_1.0.pdf). DSC-MRI analysis consisted of truncating the first 5 time points in the DSC-MRI time series to ensure steady-state magnetization, calculating the pre-bolus signal intensity on a voxel-wise basis, converting the truncated DSC-MRI time series to a concentration-time curve based on the T2* relaxivity of the contrast agent, and estimating relative tumour blood flow (rTBF) on a voxel-wise basis by using the first moment of the delta R2 (R2 = 1/T2) curves to calculate mean transit time and with leakage correction to calculate tumour blood volume as described in a previous publication (Boxerman, et al., 2006).

Antibody array pathway analysis: For the human phospho-kinase antibody array (R&D Systems), primary HUVEC (human umbilical vein endothelial cells) were cultured overnight with media supplemented with 0.75% of fetal bovine serum (FBS) followed by 15 minutes of media with 0.5%. The cells were then stimulated in serum-free media with either Apelin Pyr13 (BACHEM H-4568, 500nM), human VEGF (Peprotech, 25ng/mL), both of them or either for 5 minutes. 100 µg of cell lysate were added to the membrane and incubated overnight at 4°C with gentle agitation, following the manufacturer instructions. Films were scanned and the intensity of the bands quantified using ImageJ.

qRTPCR: Total RNA of tumours and isolated mammary epithelial cells was prepared using the RNeasy Mini Kit (Qiagen), according to the manufacturer's instructions. cDNA synthesis was performed using the iScript cDNA synthesis kit (Bio-Rad).

Bioinformatics: PolyA-mRNA of large NeuT;Apln-/- and NeuT;Apln+/+ tumours treated or untreated with sunitinib was isolated and the generated libraries were sequenced by 50-bp single-end Illumina mRNA sequencing. Differential expression analysis of aligned reads was performed with DESeq. Pathway enrichment was evaluated using GSEA (gene set enrichment analysis). Gene sets with significant enrichment were selected based on a false discovery rate (FDR) q value cut-off of 1%.

Human data: To analyse the prognostic value of apelin in cancer, we performed unbiased meta-analysis of publicly available cancer microarray datasets with clinical annotation. For breast cancer, a patient cohort of 1660 breast cancer samples was analysed using the online survival analysis tool Kaplan-Meier Plotter (Györffy, et al., 2013) - the used probe was 222856_at - and the data set GSE6532-GPL570 in the Prognoscan database (Mizuno, et al., 2009). The data set used to determine the prognostic value of apelin in NSCLC lung cancer patients was taken from GSE3141 in the Prognoscan database.

### Example 2 - Apelin as a prognostic factor

Unbiased meta-analysis of multiple datasets using the Kmplot and PrognoScan database showed that high levels of apelin expression were significantly associated with poor prognosis in breast and lung tumours (Fig. 2).

### Example 3 - Combination of apelin ablation and anti-angiogenic treatment with an RTK inhibitor

To study the role of apelin/APJ signaling and its functional significance in mammary cancer therapy *Apln*^{*-*/*-*} mice (Kuba et al., 2007) were intercrossed with MMTV-NeuT transgenic mice (Lucchini et al., 1992) to generate MMTV-NeuT;Apln^{-/-} and MMTV-NeuT;Apln^{+/+} mice (termed NeuT; *Apln*^{*-*/*-*} and NeuT; *Apln*^{*+*/*+*} hereafter). To model non-small cell lung carcinomas (NSCLC), apelin-deficient mice were crossed to a *Lox*-*Stop*-*Lox*-*KRas^{G12D}* strain together with a conditional p53 allele flanked by LoxP sites (p53^{f/f};KRas*;Apln*^{*-*/*y*} hereafter) (Jackson, 2001). Intratracheal administration of adeno-viruses expressing Cre was used to specifically induce KRas^{G12D} expression and delete p53 in pneumoyctes (DuPage et al., 2009b).

RTK inhibitors have been hampered by the onset of resistance resulting in increased leakiness of the vessels, tumour malignancy and a poor patient outcome with increased metastases (Potente et al., 2011). To study whether apelin blockade could potentiate current anti-angiogenic therapies with RTK inhibitors, the multimodal RTK inhibitor sunitinib malate was administered to NeuT; *Apln*^{*-*/*-*} or NeuT; *Apln*^{*+*/*+*} mice (Fig. 3a) and p53^{f/f};KRas;*Apln*^{*+*/*y*} or pS3^{f/f};KRas;*Apln*^{*-*/*y*} mice (Fig. 4a) upon mammary or lung tumour onset, respectively. Strikingly, apelin ablation combined with sunitinib malate treatment significantly increased the overall survival compared to either of them alone in both models (Figs. 3b and 4b). Assessment of age-matched samples of both NeuT-induced breast cancer and p53^{f/f};KRas-induced NSCLC showed a significant reduction of tumoural tissue when the mice were treated with a combination of apelin inhibition and RTK inhibition (Figs. 3c and 4c).

### Example 4 - Combination of apelin ablation and anti-angiogenic treatment with an RTK inhibitor induced tumour vessel normalisation

Analysis of tumour angiogenesis using immunohistochemical (IHC) CD31 staining and quantification of tumour blood volume overtime by non-invasive magnetic resonance imaging (MRI) showed that apelin ablation alone or treatment with the RTK inhibitor sunitinib alone reduced mammary tumour angiogenesis (Fig. 5a and b). However, unexpectedly, combination of both apelin targeting and sunitinib treatment did not further diminish tumour angiogenesis. To further understand which pathways apelin and VEGF regulate, pathway analysis by phospho-antibody array in primary human endothelial cells (HUVECs) was performed untreated or stimulated with apelin-13, recombinant VEGF or both of them together (Fig. 5a). This analysis showed that both apelin and VEGF regulate similar pathways in endothelial cells. Among these pathways, several proteins such as FAK, b-Catenin, PYK2, Src or ERK1/2 have been implicated in cell adhesion and endothelial cell permeability (Fig. 6a). VEGF-induced vessel permeability has been linked to tumour malignancy, extravasation and metastasis (Sulzmaier et al., 2014). It was aimed to further explore whether apelin ablation together with sunitinib administration had an impact on blood vessel leakage. In order to perform *in vivo* overtime studies, vessel leakage was monitored by MRI in NeuT; *Apln*^{*-*/*-*} and NeuT;*Apln*^{*+*/*+*} mammary tumours treated or untreated with sunitinib. Strikingly, these studies showed that apelin ablation reduced the sunitinib-induced vessel leakage and interstitial pressure, which was increased in more malignant tumours (Fig. 7). These results were confirmed in p53^{f/f};KRas;*Apln*^{*+*/*y*} and p53^{f/f};KRas;*Apln*^{*-*/*y*} mice by quantification of the amount of vessels with extravasated dextran within the lung tumours (Fig. 8).

### Example 5 - Combination of apelin ablation and anti-angiogenic treatment with an RTK inhibitor reduced tumour proliferation, malignancy, EMT and invasiveness

Assessment of large NeuT;*Apln*^{*-*/*-*} and NeuT;*Apln*^{*+*/*+*} tumours treated or untreated with sunitinib, showed that only sunitinib-treated NeuT;*Apln*^{-/-} mammary tumours exhibited significantly less proliferation than wild-type tumour cells (Fig. 9a). As a malignant tumour microenvironment induces EMT and invasion, we also performed mRNA analysis for EMT markers in large NeuT tumours. By qRT-PCR, it was found that several markers of EMT, metastasis, tumour angiogenesis and invasion (i.e. Snail, FN1, TgfB, Met, IL1b, Co11A1 and Co11A2) were expressed at very low levels in sunitinib-treated NeuT;Apln^{-/-} mammary tumours (Fig. 9b). Transcriptome analysis of these tumours further showed a significant enrichment of genes that correlate with stabilization and expansion of the E-cadherin-positive adherens junction, an epithelial marker, in NeuT;*Apln*^{*-*/*-*} mammary tumours treated with sunitinib compared to the other groups in gene set enrichment analysis (GSEA) (Fig. 9c). Moreover, the GSEA analysis also revealed a signature of genes positively correlated with sensitivity of breast cancer cells to dasatinib in the combinatory scenario compared to the other groups as well as with expression signatures that predict sensitivity to individual chemotherapeutic drugs (e.g. etoposide) (Figs. 9d and 9e). Importantly, a more invasive tumour microenvironment in cancer was correlated with resistance to anti-angiogenic therapy and decreased chemotherapy delivery, and, as shown above, the inventive treatment decreased the invasiveness profile of the tumour.

### Example 6 - Targeting apelin reduced metastasis induced by anti-angiogenic treatment with an RTK inhibitor

To study the impact of apelin blockage in anti-angiogenic treatment-induced metastasis, an orthotopic model of metastatic mammary cancer was used (E0771 cells). E0771 cells express apelin and APJ, and both were highly induced in tumours. As in the NeuT mammary model, orthotopically injected control cells in B6 wild-type mice (B6 Apln+/+; E0771shRen) grow significantly faster than apelin RNA-interfered cells injected into B6 Apln-/- mice (B6 Apln-/-; E0771shApln). Similarly, sunitinib treatment reduced the growth of the E0771 tumour cells in vivo (Fig. 10a). Remarkably, histological analysis of metastatic foci in the lungs from mice sacrificed with size-matched tumours, showed that apelin blockage significantly reduced anti-angiogenic sunitinib treatment-induced metastasis.

### Example 7 - Treatment of human patients

A) A female patient is diagnosed with a malignant mammary tumour of stage IIIA and grade 3. Oncogene status of the tumour is: estrogen-receptor negative, progesterone-receptor negative and HER2-positive (3+ as assessed by immunohistochemistry). The tumour responds only insufficiently to neoadjuvant treatment with trastuzumab, despite remaining HER2-positive. Therefore, the inventive treatment regimen is started (overall duration: 10 months): The patient is orally administered 50 mg sunitinib malate daily, on a four weeks on, two weeks off dosing schedule. After the first two weeks, a single intravenous dose (210 mg corresponding to 3 mg/kg body weight) of humanized monoclonal anti-apelin-receptor antibody H2aM9232N is administered. The antibody treatment is repeated every two weeks at a lower dose of 105 mg corresponding to 1.5 mg/kg body weight. After 2 months from the first day of the inventive treatment, etoposide (50mg per day for 5 days, repeated every 4 weeks) is added to the regimen. The inventive treatment inhibits the formation of metastases. After 6 months, tumour volume is reduced sufficiently for allowing removal of the tumour by breast-conserving surgery.
B) A male patient is diagnosed with a malignant non-small-cell lung cancer of stage IIIB. The oncogene status of the tumour is p53-negative, KRas-positive, assessed by immunohistochemistry. The patient is not a surgical candidate. The patient is prescribed the inventive treatment regimen, specifically a concurrent cytotoxic chemotherapy/radiotherapy regimen with an angiogenesis inhibitor and an apelin antagonist as an adjuvant therapy. Carboplatin AUC 2 IV is administered weekly for 7 weeks, and paclitaxel is given during the same 7 weeks at 50 mg/m2 IV on a weekly basis. External beam radiotherapy is begun at the same time as the chemotherapy and is delivered 5 days a week, also for 7 weeks. The patient is also orally administered 5 mg of the RTK inhibitor axitinib twice daily on an on-going continuous schedule that continues as a monotherapy after the completion of the radiochemotherapy treatment. Two weeks later, an antibody treatment is begun with a single intravenous dose of 3 mg/kg body weight of humanized monoclonal anti-apelin antibody specific for the epitope PRLSHKG (SEQ ID NO: 12) disclosed in WO 2013/012855 A1, to be repeated on a twice weekly basis at 1.5 mg/kg body weight. The size of the tumour is reduced sufficiently for the patient to be now considered a surgical candidate.

### References

Almagro et al. "Humanization of antibodies." Front Biosci 13.1 (2008): 1619-1633.
Alexiadou, et al. "Differences in plasma apelin and visfatin levels between patients with type 1 diabetes mellitus and healthy subjects and response after acute hyperglycemia and insulin administration." Hormones (Athens) 11.4 (2012): 444-50.
Angelova, et al. "Apelin and Testosterone Levels in Men with Metabolic Syndrome." Open Journal of Endocrine and Metabolic Diseases 2014 (2014).
Apte, et al. "The involvement of IL-1 in tumorigenesis, tumor invasiveness, metastasis and tumor-host interactions." Cancer and Metastasis Reviews 25.3 (2006): 387-408.
Bergers et al. Modes of resistance to anti-angiogenic therapy. Nature Reviews Cancer 8.8 (2008): 592-603.
Berta, et al., 2010. Apelin expression in human non-small cell lung cancer: role in angiogenesis and prognosis. J Thorac Oncol 5, 1120-1129.
Bhargava, et al. "Development of second-generation VEGFR tyrosine kinase inhibitors: current status." Current oncology reports 13.2 (2011): 103-111.
Birch, et al. "Antibody production." Advanced drug delivery reviews 58.5 (2006): 671-685.
Boxerman, et al. (2006). Relative cerebral blood volume maps corrected for contrast agent extravasation significantly correlate with glioma tumor grade, whereas uncorrected maps do not. AJNR. American Journal of Neuroradiology, 27(4), 859-867.
Bukowski, et al. "Third generation tyrosine kinase inhibitors and their development in advanced renal cell carcinoma." Front Oncol 2 (2012): 13.
Chapman, et al. "The apelin receptor: physiology, pathology, cell signalling, and ligand modulation of a peptide-activated class A GPCR 1." Biochemistry and cell biology 92.6 (2014): 431-440.
Chaves-Almagro, et al. "Apelin receptors: From signaling to antidiabetic strategy." European journal of pharmacology 763 (2015): 149-159.
Chow, et al. "Sunitinib: from rational design to clinical efficacy." Journal of clinical oncology 25.7 (2007): 884-896. Cox, et al., 2006. Apelin, the ligand for the endothelial G-protein-coupled receptor, APJ, is a potent angiogenic factor required for normal vascular development of the frog embryo. Developmental Biology 296, 177-189.
del Toro, et al., 2010. Identification and functional analysis of endothelial tip cell-enriched genes. Blood 116, 4025-4033. DuPage, et al. 2009a. Conditional mouse lung cancer models using adenoviral or lentiviral delivery of Cre recombinase. Nat Protoc 4, 1064-1072.
DuPage, et al., 2009b. Conditional mouse lung cancer models using adenoviral or lentiviral delivery of Cre recombinase. Nat Protoc 4, 1064-1072.
Eisenhauer, et al. "New response evaluation criteria in solid tumours: revised RECIST guideline (version 1.1)." European journal of cancer 45.2 (2009): 228-247.
Ewens, et al. (2005). Distant metastasis from subcutaneously grown E0771 medullary breast adenocarcinoma. Anticancer Research, 25(6B), 3905-3915.
Gerbier, et al. "New structural insights into the apelin receptor: identification of key residues for apelin binding." The FASEB Journal 29.1 (2015): 314-322.
Gherardi, et al. "Targeting MET in cancer: rationale and progress." Nature Reviews Cancer 12.2 (2012): 89-103.
Goel, et al. "P-Rex1 Promotes Resistance to VEGF/VEGFR-Targeted Therapy in Prostate Cancer." Cell reports 14.9 (2016): 2193-2208.
Graeven, et al., 2001. Modulation of angiogenesis and tumourigenicity of human melanocytic cells by vascular endothelial growth factor and basic fibroblast growth factor. Cancer Research 61, 7282-7290.
Györffy, et al. (2013). Online survival analysis software to assess the prognostic value of biomarkers using transcriptomic data in non-small-cell lung cancer. PloS One, 8(12), e82241.
Hammond, et al. "American Society of Clinical Oncology/College of American Pathologists guideline recommendations for immunohistochemical testing of estrogen and progesterone receptors in breast cancer (unabridged version)." Archives of pathology & laboratory medicine 134.7 (2010): e48-e72.
Hanahan & Weinberg, 2011. Hallmarks of Cancer: The Next Generation. Cell 144, 646-674.
Harvey, et al. "Estrogen receptor status by immunohistochemistry is superior to the ligand-binding assay for predicting response to adjuvant endocrine therapy in breast cancer." Journal of Clinical Oncology 17.5 (1999): 1474-1474.
Ho-Yen, et al. "The clinical and functional significance of c-Met in breast cancer: a review." Breast Cancer Research 17.1 (2015): 52.
Incio, et al. "Abstract LB-203: Obesity promotes resistance to anti-VEGF therapy in breast cancer via pro-inflammatory and angiogenic pathways." Cancer Research 75.15 Supplement (2015): LB-203.
Kalluri & Zeisberg. "Fibroblasts in cancer." Nature Reviews Cancer 6.5 (2006): 392-401.
Jackson, 2001. Analysis of lung tumour initiation and progression using conditional expression of oncogenic K-ras. Genes Dev. 15, 3243-3248.
Jayson, et al. "Antiangiogenic therapy in oncology: current status and future directions." The Lancet (2016), doi:10.1016/S0140-6736(15)01088-0
Jeltsch, et al. "Receptor tyrosine kinase-mediated angiogenesis." Cold Spring Harbor perspectives in biology 5.9 (2013): a009183.
Kasai, A., et al. 2008. Retardation of Retinal Vascular Development in Apelin-Deficient Mice. Arteriosclerosis, Thrombosis, and Vascular Biology 28, 1717-1722.
Kasai, A., et al., 2004. Apelin is a novel angiogenic factor in retinal endothelial cells. Biochemical and Biophysical Research Communications 325, 395-400.
Kawamata, et al. "Molecular properties of apelin: tissue distribution and receptor binding." Biochimica et Biophysica Acta (BBA)-Molecular Cell Research 1538.2 (2001): 162-171.
Kälin, et al. 2007. Paracrine and autocrine mechanisms of apelin signaling govern embryonic and tumour angiogenesis. Developmental Biology 305, 599-614.
Kidoya, et al., 2012. Biology of the apelin-APJ axis in vascular formation. J Biochem.
Kidoya, et al., 2008. Spatial and temporal role of the apelin/APJ system in the caliber size regulation of blood vessels during angiogenesis. EMBO J 27, 522-534.
Kuba, et al., 2007. Impaired Heart Contractility in Apelin Gene Deficient Mice Associated With Aging and Pressure Overload. Circulation Research 101, e32-e42.
Lamouille, et al. "Molecular mechanisms of epithelial-mesenchymal transition." Nature reviews. Molecular cell biology 15.3 (2014): 178.
Larsen, et al. "Improved method for predicting linear B-cell epitopes." Immunome research 2.1 (2006): 1.
Lee, et al. "Modification of the terminal residue of apelin-13 antagonizes its hypotensive action." Endocrinology 146.1 (2005): 231-236.
Lucchini, et al, 1992. Early and multifocal tumours in breast, salivary, harderian and epididymal tissues developed in MMTY-Neu transgenic mice. Cancer Lett. 64, 203-209.
Macaluso, et al. "Discovery of a competitive apelin receptor (APJ) antagonist." ChemMedChem 6.6 (2011): 1017-1023.
Maloney, et al. "Discovery of 4-oxo-6-((pyrimidin-2-ylthio) methyl)-4H-pyran-3-yl 4-nitrobenzoate (ML221) as a functional antagonist of the apelin (APJ) receptor." Bioorganic & medicinal chemistry letters 22.21 (2012): 6656-6660.
Martin TA, et al. Cancer Invasion and Metastasis: Molecular and Cellular Perspective. In: Madame Curie Bioscience Database [Internet]. Austin (TX): Landes Bioscience; 2000-2013. Available from: http://www.ncbi.nlm.nih.gov/books/NBK164700/
Mizuno, H., et al. (2009). PrognoScan: a new database for meta-analysis of the prognostic value of genes. BMC Medical Genomics, 2 (1) , 18.
Muller, et al., 1988. Single-step induction of mammary adenocarcinoma in transgenic mice bearing the activated c-neu oncogene. Cell 54, 105-115.
Phan, Vernon T., et al. "Oncogenic RAS pathway activation promotes resistance to anti-VEGF therapy through G-CSF-induced neutrophil recruitment." Proceedings of the National Academy of Sciences 110.15 (2013): 6079-6084.
Potente, et al., 2011. Basic and Therapeutic Aspects of Angiogenesis. Cell 146, 873-887.
Puffer, et al. "Expression and coreceptor function of APJ for primate immunodeficiency viruses." Virology 276.2 (2000): 435-444.
Rakha, et al. "Updated UK Recommendations for HER2 assessment in breast cancer." Journal of Clinical Pathology 68.2 (2015): 93. Rayalam, et al. "A putative role for apelin in the etiology of obesity." Biochemical and biophysical research communications 368.3 (2008): 815-819.
Reaux, et al. "Distribution of apelin-synthesizing neurons in the adult rat brain." Neuroscience 113.3 (2002): 653-662. Roskoski. "Src protein-tyrosine kinase structure, mechanism, and small molecule inhibitors." Pharmacological Research 94 (2015): 9-25.
Saint-Geniez et al., 2002. Expression of the murine msr/apj receptor and its ligand apelin is upregulated during formation of the retinal vessels. Mech. Dev. 110, 183-186.
Schenone, et al. "SRC kinase inhibitors: An update on patented compounds." Current medicinal chemistry 18.33 (2011): 5061-5078. Sorli, et al., 2007. Apelin is a potent activator of tumour neoangiogenesis. Oncogene 26, 7692-7699.
Sulzmaier, et al. "FAK in cancer: mechanistic findings and clinical applications." Nature Reviews Cancer 14.9 (2014): 598-610.
Sun, et al. "Design, synthesis, and evaluations of substituted 3-[(3-or 4-carboxyethylpyrrol-2-yl) methylidenyl] indolin-2-ones as inhibitors of VEGF, FGF, and PDGF receptor tyrosine kinases." Journal of medicinal chemistry 42.25 (1999): 5120-5130.
Tatemoto, et al., 1998. Isolation and characterization of a novel endogenous peptide ligand for the human APJ receptor. Biochemical and Biophysical Research Communications 251, 471-476.
Van Cutsem, et al. "Cetuximab and chemotherapy as initial treatment for metastatic colorectal cancer." New England Journal of Medicine 360.14 (2009): 1408-1417.
Zhang, Yu, et al. "Low plasma apelin in newly diagnosed type 2 diabetes in Chinese people." Diabetes Care 32.12 (2009): e150-e150.
Zhou, et al. "Binding of ALX40-4C to APJ, a CNS-based receptor, inhibits its utilization as a co-receptor by HIV-1." Virology 312.1 (2003): 196-203.

All references to patents and patent applications cited herein and the above references are included herein by reference in their entirety.

## Claims

1. A pharmaceutical composition comprising an apelin antagonist and/or an apelin receptor antagonist, and further comprising an angiogenesis inhibitor, wherein the angiogenesis inhibitor is selected from the group of receptor tyrosine kinase (RTK) inhibitors.

2. A set comprising a first pharmaceutical composition, comprising an apelin antagonist and/or an apelin receptor antagonist, and a second pharmaceutical composition, comprising an angiogenesis inhibitor, wherein the angiogenesis inhibitor is selected from the group of RTK inhibitors.

3. The composition or set according to claim 1 or 2, wherein the angiogenesis inhibitor is selected from the group of sunitinib, sorafenib, pazopanib, axitinib, cabozantinib, regorafenib, vandetanib, tivozanib, cediranib, nintedanib and vatalanib.

4. The composition or set according to claim 3, wherein the angiogenesis inhibitor is sunitinib.

5. The composition or set according to any one of claims 1 to 4, wherein the apelin antagonist is selected from the group of anti-apelin antibodies, and/or
wherein the apelin receptor antagonist is selected from apelin analogues, preferably a peptide with the sequence QRPRLSHKGPMPA (SEQ ID NO: 14) or N-α-acetyl-nona-D-arginine amide (SEQ ID NO: 22) or cyclo(1-6)CRPRLC-KH-cyclo(9-14)CRPRLC (SEQ ID NO: 23), and anti-apelin-receptor antibodies.

6. An apelin antagonist and/or apelin receptor antagonist for use in the prevention or treatment of cancer in a patient, wherein the apelin antagonist and/or apelin receptor antagonist is administered to the patient, and
wherein an angiogenesis inhibitor, preferably selected from the group of RTK inhibitors, is administered to the patient, in a composition together with the apelin antagonist and/or apelin receptor antagonist, in combination with the apelin antagonist and/or apelin receptor antagonist, before the apelin antagonist and/or apelin receptor antagonist is administered, or after the apelin antagonist and/or apelin receptor antagonist has been administered;
preferably wherein the angiogenesis inhibitor is selected from the group of sunitinib, sorafenib, pazopanib, axitinib, cabozantinib, regorafenib, vandetanib, tivozanib, cediranib, nintedanib and vatalanib; especially sunitinib.

7. An apelin antagonist and/or apelin receptor antagonist for use in the treatment of a cancer patient who has resistance or is at risk of developing resistance to anti-angiogenic therapy, preferably anti-angiogenic therapy with an angiogenesis inhibitor selected from the group of RTK inhibitors;
preferably wherein the angiogenesis inhibitor is selected from the group of sunitinib, sorafenib, pazopanib, axitinib, cabozantinib, regorafenib, vandetanib, tivozanib, cediranib, nintedanib and vatalanib.

8. The apelin antagonist and/or apelin receptor antagonist for use according to claim 6 or 7, wherein the angiogenesis inhibitor is sunitinib.

9. The apelin antagonist and/or apelin receptor antagonist for use according to any one of claims 6 to 8, wherein the cancer is selected from breast cancer and lung cancer.

10. The apelin antagonist and/or apelin receptor antagonist for use according to any one of claims 6 to 9, wherein the cancer is HER-2-positive, estrogen-receptor-positive, p53-negative, KRaspositive or combinations thereof.

11. The apelin antagonist and/or apelin receptor antagonist for use according to any one of claims 6 to 10, wherein the cancer over-expresses epithelial-mesenchymal transition (EMT) markers, preferably selected from the group of Snail, fibronectin, and TGF-beta, and/or
wherein the cancer over-expresses invasiveness-related genes, preferably selected from the group of Met, IL1b, COL1A1 and COL1A2 and/or
wherein the cancer over-expresses apelin and/or apelin receptor.

12. The apelin antagonist and/or apelin receptor antagonist for use according to any one of claims 6 to 11, wherein a further chemotherapeutic agent, and/or radiotherapy for the latter three of the following cases, is administered to the patient, in a composition together with the apelin antagonist and/or apelin receptor antagonist, in combination with the apelin antagonist and/or apelin receptor antagonist, before the apelin antagonist and/or apelin receptor antagonist is administered, or after the apelin antagonist and/or apelin receptor antagonist has been administered,
wherein the further chemotherapeutic agent is selected from the group of alkylating agents, platin-based chemotherapeutic agents, antimetabolites, anti-microtubule agents, topoisomerase inhibitors and cytotoxic antibiotics,
preferably wherein the further chemotherapeutic agent is selected from the group of 5-fluorouracil, cisplatin, carboplatin, cyclophosphamide, doxorubicin, epirubicin, gemcitabine, taxanes such as paclitaxel or docetaxel, methotrexate, pemetrexed, vinorelbine and etoposide, in particular etoposide.

13. The apelin antagonist and/or apelin receptor antagonist for use according to any one of claims 6 to 12, wherein a Src tyrosine kinase inhibitor is also administered to the patient, in a composition together with the apelin antagonist and/or apelin receptor antagonist, in combination with the apelin antagonist and/or apelin receptor antagonist, before the apelin antagonist and/or apelin receptor antagonist is administered or after the apelin antagonist and/or apelin receptor antagonist has been administered,
preferably wherein the Src tyrosine kinase inhibitor is selected from bosutinib, dasatinib, ponatinib, vandetanib, saracatinib and AZD0424, especially dasatinib.

14. The apelin antagonist and/or apelin receptor antagonist for use according to any one of claims 6 to 13, wherein the patient has an apelin level, preferably a plasma or serum apelin level, higher than a control, and/or the cancer has an apelin or apelin receptor level higher than a control;
preferably wherein the apelin level is measured after the patient has undergone cancer treatment with an angiogenesis inhibitor, preferably selected from the group of RTK inhibitors, especially sunitinib.

15. The apelin antagonist and/or apelin receptor antagonist for use according to any one of claims 6 to 14, for tumour blood vessel normalisation.
